# EUROPEAN PATENT APPLICATION

(11) **EP 4 691 756 A1**
(43) Date of publication of application: **11.02.2026**
(21) Application number: 24780088.1
(22) Date of filing: 23.03.2024
(51) Int. Cl.: B32B 3/24, B32B 5/18, G01N 33/543

(54) **LAMINATE INCLUDING TRANSPARENT ELECTROCONDUCTIVE FILM AND POROUS FILM, BIOCHIP INCLUDING LAMINATE, AND METHOD FOR MANUFACTURING THESE**

(30) Priority: 24.03.2023 JP 2023047649
(71) Applicant: Watanabe, Takeshi, Sagamihara-shi, Kanagawa 252-5258 (JP)
(72) Inventor: KOH Shinji, Sagamihara-shi, Kanagawa 252-5258 (JP); WATANABE Takeshi, Sagamihara-shi, Kanagawa 252-5258 (JP)
(74) Representative: Wasner, Marita
(86) International application number: PCT/JP2024/011524
(87) International publication number: WO 2024/203970

(57) **Abstract**

[Problem] To provide a transparent electroconductive film that can be supplied for use in quick and simple inspection equipment, and a biochip in which the transparent electroconductive film is used. In particular, to provide a transparent electroconductive film that can be used for immunoassay utilizing electrochemical light emission, and a biochip in which the transparent electroconductive film is used.

[Solution] The present invention provides a laminate comprising a transparent electroconductive film and a porous film that is laminated on the transparent electroconductive film, wherein the porous film is permeable with respect to an electrolyte solution.

## Description

### Technical Field

The present invention relates to a laminated body comprising a transparent conductive film on which a porous membrane having micropores is laminated, a biochip comprising the laminated body, and methods for producing these. The present invention also relates to an electrochemiluminescence (ECL) immunoassay method using the laminated body and the biochip.

### Background Art

In recent years, rapid and simple testing performed near patients, known as point-of-care testing (POCT), has been considered important for disease prevention, health promotion, and infection control. In particular, triggered by the spread of COVID-19, there has been an increasing demand for the development of devices that can achieve both simple measurement and high sensitivity. A representative rapid and simple testing method, the immunochromatographic method, involves an antigen in a specimen forming an immune complex with a colloidal-gold-labeled antibody, the immune complex migrating through a nitrocellulose membrane, and the immune complex being trapped by a capture antibody to cause color development, which is then visually determined. Although this method offers excellent simplicity and rapidity, its reliance on visual judgment makes it difficult to improve sensitivity, and it is generally limited to qualitative testing.

Electrochemiluminescence (ECL) immunoassay is known as a highly sensitive immunoassay method. Electrochemiluminescence refers to a luminescence phenomenon that occurs through an electrode reaction, and unlike conventional immunoassay methods based on fluorescence or absorbance measurement, it does not require excitation or incident light. Therefore, measurement can be performed safely and inexpensively with a low noise level, enabling highly sensitive analysis. Typically, tris(2,2'-bipyridyl)ruthenium(II) is used as an electrochemiluminescent labeling reagent, and tripropylamine is used as a co-reactant. Immunoassay methods using these reagents have been reported (Patent Document 1).
The method described in Patent Document 1 involves labeling an antibody that specifically binds to a target molecule with tris(2,2'-bipyridyl)ruthenium(II), forming an immune complex through an antigen-antibody reaction, and then, in a tripropylamine solution, oxidizing tripropylamine or both tripropylamine and tris(2,2'-bipyridyl)ruthenium(II) to generate luminescence. By measuring the amount of antibody bound to the target compound, or the amount of unbound antibody, the concentration of the target molecule can be estimated.

At present, electrochemiluminescence (ECL) immunoassay has been adopted in Roche's automated clinical analyzers (Non-Patent Document 1). In these automated analyzers, an immune complex labeled with tris(2,2'-bipyridyl)ruthenium(II) is first formed on magnetic beads, and the properties of the magnetic beads are then used to fix the complex onto the surface of a platinum electrode. Using an electrolyte solution containing tripropylamine, electrochemiluminescence is generated near the platinum electrode, and the luminescence intensity is measured. However, because ECL immunoassay requires electrodes, and the above analyzers measure multiple specimens using a single electrode, a cleaning step is required between measurements of different specimens. This makes it difficult to miniaturize the device and simplify the measurement process, posing a challenge in developing measurement devices compatible with POCT.

With respect to immunoassay methods compatible with POCT, many reports exist on measurement techniques that combine immunochromatography with fluorescence method, chemiluminescence method, or chemiluminescent enzyme method. In practice, devices and kits based on fluorescence chromatography as the measurement principle have been commercialized, such as the portable immunofluorescence analyzer SPOTCHEM FLORA (ARKRAY, Inc.) and the SARS coronavirus antigen kit Exdia EK Test COVID-19 Ag (Eiken Chemical Co., Ltd.).

On the other hand, there have been no reports on measurement methods that combine electrochemiluminescence (ECL) with immunochromatography. Since the ECL method requires electrodes, a challenge arises in combining it with disposable measurement chips for immunochromatography, which are generally intended for single use, as disposable electrodes must be employed. In addition, membranes commonly used in immunochromatography have a thickness of about 100 µm, while the diameter of magnetic beads used in practical ECL immunoassay devices is about 3 µm. Such a thickness of 100 µm increases the probability that reactive species generated by the electrode reaction become deactivated before reaching the electrochemiluminescent labeling substances immobilized in the membrane, which may significantly reduce luminescence efficiency. These factors are considered to be the reasons why it has been difficult to combine immunochromatography with the ECL method.

With regard to research on POCT devices utilizing electrochemiluminescence (ECL) immunoassay, many studies have been reported using low-cost electrodes such as paper-based electrodes and screen-printed electrodes; however, none have yet reached practical use as clinical analyzers.

In developing an electrochemiluminescence (ECL) immunoassay device compatible with POCT, it is necessary to miniaturize the device itself. By utilizing a transparent conductive film as an electrode, greater flexibility in device configuration can be achieved, making miniaturization easier. However, a representative transparent conductive film, indium tin oxide (ITO), contains indium, which is a rare element, and therefore is not suitable for use as a disposable electrode. In addition, in oxide-based transparent conductive films such as ITO, oxidation reactions of organic substances such as tripropylamine are less likely to occur, thereby making it difficult to obtain high luminescence intensity in immunoassay measurement systems.

As research applying transparent conductive films free of rare elements to electrochemiluminescence (ECL), there have been reports using carbon-based transparent electrodes fabricated by pyrolyzing photoresist (Non-Patent Document 2). However, with such carbon-based films, achieving both high conductivity and high optical transparency remains a challenge.

The present inventors have previously proposed that by employing graphene, a transparent carbon material, as a disposable electrode for electrochemiluminescence (ECL) immunoassay, it is possible to construct a system suitable for rapid and simple testing. Using a graphene electrode fabricated by chemical vapor deposition (CVD), the present inventors succeeded in performing an immunoassay of carcinoembryonic antigen (CEA), which is a cancer marker, and demonstrated and reported that the graphene transparent electrode possesses excellent properties as an electrode for ECL immunoassay (Non-Patent Document 3). Research on the use of graphene electrodes for ECL immunoassay applications has not been reported by others, and this constitutes an original and unique technology.

### Prior Art Documents

### Patent Documents

Patent Document 1: Japanese Unexamined Patent Application Publication No. H10-111293

### Non-Patent Documents

Non-Patent Document 1: S. Parveen, M. S. Aslam, L. Hu, and G. Xu, Electrogenerated Chemiluminescence. Protocols and Applications, Heidelberg, 2013, pp. 39-44, ("3.4 Commercial ECL Instruments").
Non-Patent Document 2: E. K. Walker et al., "Carbon Optically Transparent Electrodes for Electrogenerated Chemiluminescence," Langmuir, 28, pp. 1604-1610 (2012). https://doi.org/10.1021/1a2042394.
Non-Patent Document 3: T. Watanabe et al., "Single-layer Graphene as a Transparent Electrode for Electrogenerated Chemiluminescence Biosensing," Electrochemistry Communications, 138, 107290 (2022). https://doi.org/10.1016/j.elecom.2022.1

### Summary of Invention

### Problems to be Solved by the Invention

An object of the present invention is to provide a transparent conductive film suitable for use in rapid and simple testing devices, as well as a biochip utilizing the same. In particular, the present invention aims to provide a transparent carbon-based conductive film applicable to immunoassays employing electrochemiluminescence, and a biochip incorporating such a film.

### Means for Solving the Problems

As described above, the present inventors had demonstrated that CVD graphene electrodes possess properties suitable for electrochemiluminescence (ECL) immunoassays compatible with rapid and simple testing. However, for use in immunoassays, the challenge of antibody modification on graphene electrodes needed to be addressed. The inventors therefore examined various methods for conjugating graphene with antibody molecules, including covalent bonding via diazonium salt reduction and π-π interactions between pyrene groups and graphene. However, in all of these methods, because graphene is a two-dimensional sheet, the amount of antibody modification was insufficient, and in covalent modification methods, the π-conjugated system of graphene was disrupted, leading to increased sheet resistance. Thus, it was not possible to achieve both immobilization of antibodies on graphene electrodes and preservation of their properties.
Accordingly, the inventors conceived of a method for immobilizing proteins onto the polymer film used as a support layer in the transfer step required for the production of CVD graphene electrodes. In conventional graphene transfer methods, polymethyl methacrylate (PMMA) has been used as a support material. In this method, after forming a PMMA film on a graphene film grown on a metal catalyst substrate, the metal catalyst substrate is etched, and the PMMA/graphene laminated body is transferred onto a desired substrate. After the transfer or before use as an electrode, the PMMA film is dissolved and removed to obtain a CVD graphene electrode. The inventors initially examined using the PMMA support film itself as a protein immobilization layer without removing it; however, when the PMMA film remained laminated on the graphene electrode, the electrolyte solution containing the ECL reagents could not sufficiently permeate the PMMA film, making electrochemical measurements impossible. Through extensive studies, the inventors conceived of using a porous membrane, instead of a PMMA film, that allows sufficient transport of ECL reactants through the membrane. By using an electrode comprising such a porous membrane and a graphene film, they succeeded in performing immunoassays based on electrochemiluminescence. The present invention has been completed based on these findings, and includes the following embodiments:

One aspect of the present invention is:
[1] A laminated body comprising a transparent conductive film and a porous membrane laminated on the transparent conductive film,
   wherein the porous membrane is permeable to an electrolyte solution.
   In one embodiment, the laminated body of the present invention is:
[2] The laminated body according to [1],
   wherein the porous membrane has pores with an average pore diameter of 20 nm or more. In one embodiment, the laminated body of the present invention is:
[3] The laminated body according to [1] or [2],
   wherein the pore area ratio on the porous membrane surface is 20% or more.
   In one embodiment, the laminated body of the present invention is:
[4] The laminated body according to any one of [1] to [3],
   wherein the porous membrane has a film thickness of 100 nm or more.
   In one embodiment, the laminated body of the present invention is:
[5] The laminated body according to any one of [1] to [4],
   further comprising a transparent insulating substrate,
   wherein the porous membrane is laminated on a first surface of the transparent conductive film, and the transparent insulating substrate is laminated on a second surface of the transparent conductive film.
   In one embodiment, the laminated body of the present invention is:
[6] The laminated body according to [5], wherein the laminated body further comprises an electrical conductor, the electrical conductor being laminated on a surface of the porous membrane opposite to the surface on which the transparent conductive film is laminated, or on a surface of the transparent insulating substrate on the same surface as the surface on which the transparent conductive film is laminated.
   In one embodiment, the laminated body of the present invention is:
[7] The laminated body according to any one of [1] to [6],
   wherein the porous membrane has a compressed structure at least in part.
   In one embodiment, the laminated body of the present invention is:
[8] The laminated body according to any one of [5] to [7],
   wherein the transparent insulating substrate is coated with a transparent conductive additive.
   In one embodiment, the laminated body of the present invention is:
[9] The laminated body according to any one of [1] to [4],
   further comprising an electrical conductor,
   wherein the transparent conductive film is laminated on a first surface of the porous membrane, and the electrical conductor is laminated on a second surface of the porous membrane.
   In one embodiment, the laminated body of the present invention is:
[10] The laminated body according to any one of [1] to [9],
   wherein the porous membrane is a membrane made of a material selected from the group consisting of nitrocellulose, cellulose acetate, polyethersulfone, polytetrafluoroethylene, polyamide, polyvinylidene fluoride, regenerated cellulose, polycarbonate, polypropylene, polyvinylidene chloride, aluminum oxide, glass fibers, quartz fibers, polymethyl methacrylate, polystyrene, polyethylene, polyethylene terephthalate, and ceramics, or a mixed membrane made of two or more materials selected from the group.
   In one embodiment, the laminated body of the present invention is:
[11] The laminated body according to any one of [1] to [10],
   wherein the transparent conductive film is a carbon-based conductive film.
   In one embodiment, the laminated body of the present invention is:
[12] The laminated body according to any one of [1] to [11],
   wherein the transparent conductive film is a graphene film.

### Another aspect of the present invention is:

[13] A biochip comprising the laminated body according to any one of [5] to [12]. Another aspect of the present invention is:

[14] A kit comprising the biochip according to [13] and an electrochemiluminescent substance.

### Another aspect of the present invention is:

[15] The laminated body according to [5], further comprising a transparent conductive film, a transparent insulating substrate, and an electrical conductor,
wherein the laminated body is stacked in the order of a transparent insulating substrate, a transparent conductive film, a porous membrane, a transparent conductive film, and a transparent insulating substrate,
and wherein the electrical conductor is disposed so as to be in contact with the transparent conductive film.

### Another aspect of the present invention is:

[16] A method of using the laminated body according to [5] as a transparent electrode, wherein a solvent having the same refractive index as the porous membrane is used. Another aspect of the present invention is:

[17] A method for detecting a target compound by electrochemiluminescence immunoassay using the laminated body according to any one of [5] to [12] or the biochip according to [13], comprising:
(a) binding the target compound with an electrochemiluminescent substance;
(b) capturing the target compound in the porous membrane of the laminated body with a specific binding substance for the target compound immobilized in the porous membrane; and
(c) after steps (a) and (b), applying a potential to the laminated body and measuring electrochemiluminescence.

### Another aspect of the present invention is:

[18] A method for producing a laminated body in which a transparent insulating substrate, a carbon-based conductive film, and a porous membrane are laminated in this order, comprising:
(a) forming a carbon-based conductive film on a metal catalyst substrate by a chemical vapor deposition (CVD) method;
(b) further forming a porous membrane on the carbon-based conductive film to prepare a laminated body; and
(c) removing the metal catalyst substrate by etching and transferring the laminated body so that the carbon-based conductive film contacts a transparent insulating substrate.

In one embodiment, the method for producing a laminated body of the present invention is:
The method for producing a laminated body according to [18], wherein, in step (b), the solvent used to form the porous membrane is a mixed solvent comprising a good solvent and a poor solvent for the polymer used therein.

### Effects of the Invention

According to the laminated body comprising a transparent conductive film, a porous membrane, and a transparent insulating substrate of the present invention, proteins such as antibodies can be immobilized on the porous membrane, thereby enabling electrochemiluminescence immunoassay using the laminated body as an electrode. Since the laminated body comprising a transparent conductive film, a porous membrane, and a transparent insulating substrate does not contain rare elements, it can be manufactured at low cost and used as a disposable electrode, making it suitable for rapid and simple testing devices.

Furthermore, when the laminated body of the present invention is used for electrochemiluminescence immunoassay, it allows efficient detection of luminescence generated within the porous membrane without attenuation, thereby contributing to enhanced sensitivity of rapid and simple testing devices.

### Brief Description of Drawings

**[****Fig. 1]** Fig. 1 is a schematic diagram showing one embodiment of the laminated body according to the present invention. The laminated body comprises a porous membrane and a transparent conductive film.
**[****Fig. 2]** Fig. 2 is a schematic diagram showing one embodiment of the laminated body according to the present invention. The laminated body comprises a porous membrane, a transparent conductive film, and a transparent insulating substrate.
**[****Fig. 3]** Fig. 3 is a schematic diagram showing one embodiment of the biochip according to the present invention.
**[****Figs. 4A and 4B]** Figs. 4A and 4B are schematic diagrams showing a modification of one embodiment of the biochip according to the present invention.
**[****Figs. 4C and 4D]** Figs. 4C and 4D are schematic diagrams showing a modification of one embodiment of the biochip according to the present invention.
**[****Fig. 4E]** Fig. 4E is a schematic diagram showing a modification of one embodiment of the biochip according to the present invention.
**[****Fig. 4F]** Fig. 4F is a schematic diagram showing a modification of one embodiment of the biochip according to the present invention.
**[****Fig. 4G]** Fig. 4G is a schematic diagram showing a modification of one embodiment of the biochip according to the present invention.
**[****Fig. 4H]** Fig. 4H is a schematic diagram showing a modification of one embodiment of the biochip according to the present invention.
**[****Fig. 5]** Fig. 5 is a schematic diagram illustrating one embodiment of a method for producing a laminated body according to the present invention.
**[****Fig. 6]** Fig. 6 is a schematic diagram illustrating a conventional method for producing a CVD graphene electrode including a transfer process.
**[****Fig. 7]** Fig. 7 shows SEM images of the porous membrane surface of a porous membrane/graphene laminated body produced in Example 1 below.
**[****Fig. 8]** Fig. 8 shows histograms of pore diameters on the porous membrane surface of a porous membrane/graphene laminated body produced in Example 1 below.
**[****Fig. 9]** Fig. 9 shows histograms of pore diameters on the porous membrane surface of a porous membrane/graphene laminated body produced in Example 1 below.
**[****Fig. 10]** Fig. 10 is a schematic diagram of the electrochemiluminescence measurement cell used in Example 4 below.
**[****Fig. 11]** Fig. 11 shows photographs of a porous membrane/graphene film/transparent insulating substrate laminated body in the electrochemical measurement cell in Example 4 (Fig. 11A) and a photograph during electrochemiluminescence generation (Fig. 11B).
**[****Fig. 12]** Fig. 12 shows the results of electrochemiluminescence measurement conducted by cyclic voltammetry (CV) using the porous membrane/graphene film/transparent insulating substrate laminated body as a working electrode in Example 4 below.
**[****Fig. 13]** Fig. 13 is a schematic diagram of a three-electrode measurement chip used for electrochemiluminescence measurement in Example 5 below.
**[****Fig. 14]** Fig. 14 shows the results of electrochemiluminescence measurement using the laminated body of the present invention as a three-electrode measurement chip in Example 5 below.
**[****Fig. 15]** Fig. 15 shows the results of immunoassay based on electrochemiluminescence using the laminated body of the present invention in Example 6 below.
**[****Fig. 16]** Fig. 16 shows bar graphs of ECL intensity when electrochemiluminescence (ECL) measurements were repeatedly performed using a cellulose membrane/graphene film laminated body according to the present invention in Example 7 (left), and for comparison, the results of ECL measurement using a graphene electrode (right).
**[****Fig. 17]** Fig. 17 shows SEM images of the porous membrane surface of a porous membrane/graphene laminated body produced by a solution-casting method in Example 8 below. Fig. 17(a) shows an image when the porous membrane was formed using acetone as a solvent, and Fig. 17(b) shows an image when the porous membrane was formed using an acetone/formamide mixed solvent.
**[****Fig. 18]** Fig. 18 shows SEM images of the porous membrane surfaces of six types of porous membrane/graphene laminated bodies produced by a solution-casting method in Example 8 below. The conditions of mixed cellulose ester solution concentration and immersion in ultrapure water for Figs. 18(a)-(f) are as follows: (a) 4 wt% without immersion; (b) 4 wt% with immersion; (c) 8 wt% without immersion; (d) 8 wt% with immersion; (e) 12 wt% without immersion; (f) 12 wt% with immersion.
**[****Fig. 19]** Fig. 19 shows a photograph of a porous mixed cellulose ester membrane/graphene film/quartz glass substrate laminated body produced in Example 8 below.
**[****Fig. 20]** Fig. 20(a) shows a Raman spectrum of a porous mixed cellulose ester membrane/graphene film/quartz glass substrate laminated body produced in Example 8 below. Fig. 20(b) shows a Raman spectrum of a graphene film on a quartz glass substrate after removal of the porous mixed cellulose ester membrane.
**[****Fig. 21]** Fig. 21 shows the results of electrochemiluminescence measurement of a porous mixed cellulose ester membrane/graphene film/quartz glass substrate laminated body produced in Example 8 below. Fig. 21(a) shows a current-potential curve, and Fig. 21(b) shows an ECL intensity-potential curve.
**[Fig. 22]** Fig. 22 is a schematic diagram showing the production process of a porous cellulose membrane/graphene film laminated body produced by a phase inversion method in Example 9 below.
**[****Fig. 23]** Fig. 23 shows SEM images of the cellulose membrane in a porous cellulose membrane/graphene film/copper foil laminated body produced in Example 9 below. Fig. 23(a) shows an SEM image when the applicator gap height during cellulose membrane formation in the laminated body was 25 µm. Figs. 23(b)-(d) respectively show SEM images when the applicator gap heights during cellulose membrane formation were 50 µm, 75 µm, and 100 µm.
**[****Fig. 24]** Fig. 24 is a schematic diagram showing the method for measuring protein adsorption amount conducted in Example 9 below.
**[****Fig. 25]** Fig. 25 is a calibration curve prepared based on the results of protein adsorption amount measurement conducted in Example 9 below.
**[****Fig. 26]** Fig. 26 shows the quantitative results of protein adsorption amount measured using four types of cellulose membranes and a commercial nitrocellulose membrane in Example 9 below.
**[****Fig. 27]** Fig. 27 is a schematic diagram of a compression method of a porous structure using a hydraulic flask press conducted in Example 10 below.
**[****Fig. 28]** Fig. 28 is a photograph of a porous mixed cellulose ester membrane/graphene film laminated body on a quartz glass substrate with gold/chromium deposited at both ends, used in Example 10 below.
**[****Fig. 29]** Fig. 29 shows photographs before and after compression of a porous mixed cellulose ester membrane/graphene film laminated body on a quartz glass substrate using an aluminum block with a hole of 4 mm diameter in Example 10 below (left: before compression; right: after compression).
**[****Fig. 30]** Fig. 30 shows SEM images of the cellulose surface of a laminated body having a partially compressed porous structure produced in Example 10 below. Fig. 30(a) shows an SEM image of the compressed porous structure portion, and Fig. 30(b) shows an SEM image of the uncompressed porous structure portion.
**[****Fig. 31]** Fig. 31 shows the results of electrochemiluminescence measurement of a porous mixed cellulose ester membrane/graphene film/quartz glass substrate laminated body having a partially compressed porous structure produced in Example 10 below (solid line). For comparison, the measurement results of a porous mixed cellulose ester membrane/graphene film/quartz glass substrate laminated body without a compressed porous structure are also shown (dashed line).
**[****Fig. 32]** Fig. 32 shows a current-potential curve obtained from ECL immunoassay using a porous mixed cellulose ester membrane/graphene film/quartz glass substrate laminated body produced in Example 11 below.
**[****Fig. 33]** Fig. 33 shows an ECL intensity-potential curve obtained from ECL immunoassay using a porous mixed cellulose ester membrane/graphene film/quartz glass substrate laminated body produced in Example 11 below.
**[****Fig. 34]** Fig. 34 shows transmittance measurement results (left) and Raman spectra (right) of graphene/PEDOT:PSS electrodes, graphene electrodes, and PEDOT:PSS electrodes in Example 12 below.
**[****Fig. 35]** Fig. 35 shows the results of electrochemical measurement by CV using graphene, graphene/PEDOT:PSS, and glassy carbon as electrodes in Example 12 below.
**[****Fig. 36]** Fig. 36(a) shows ECL intensity-potential curves obtained from ECL measurements using graphene/PEDOT:PSS electrodes, graphene electrodes, and PEDOT:PSS electrodes in Example 12 below. Fig. 36(b) shows a graph of the total luminescence for 30 seconds from the start of potential application obtained by chronoamperometry.
**[****Fig. 37]** Fig. 37 shows electrochemical measurement results by CV of Ru(bpy)₃Cl₂ (Fig. 37(a)) or tripropylamine (Fig. 37(b)) using graphene or graphene/PEDOT:PSS as electrodes in Example 12 below.
**[****Fig. 38]** Fig. 38 shows the relationship between refractive index and glycerol ratio in water/glycerol mixed solvents measured in Example 13 below.
**[****Fig. 39]** Fig. 39 shows transmittance spectra of porous PVDF membranes in glycerol/water mixed solutions with various ratios measured in Example 13 below.
**[****Fig. 40]** Fig. 40 shows transmittance spectra of a PVDF/graphene/quartz glass substrate laminated body in water/glycerol solutions measured in Example 13 below.
**[****Fig. 41]** Fig. 41 is a schematic diagram of a film-type electrochromic device produced in Example 13 below.
**[****Fig. 42]** Fig. 42 shows a current-voltage curve of a film-type electrochromic device produced in Example 13 below.
**[****Fig. 43]** Fig. 43 is a set of photographs showing reversible color changes between colored and decolored states of a film-type electrochromic device produced in Example 13 below.

### Modes for Carrying Out the Invention

One aspect of the present invention provides a laminated body comprising a transparent conductive film and a porous membrane laminated on the transparent conductive film, wherein the porous membrane is permeable to an electrolyte solution.

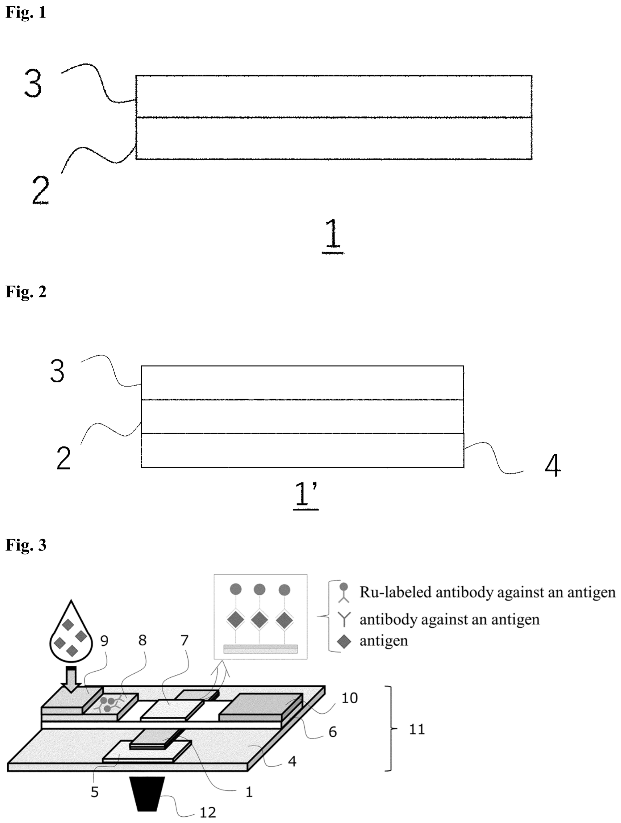

An embodiment of the laminated body according to this aspect is shown in Fig. 1. As shown in Fig. 1, the laminated body 1 is composed of a transparent conductive film 2 and a porous membrane 3.

The transparent conductive film 2 is a film having both high electrical conductivity and visible light transmittance. Examples of the transparent conductive film 2 that can be used in the present invention include, but are not limited to, indium tin oxide, fluorine-doped tin oxide, zinc oxide, tin oxide, titanium oxide, metal nanowires such as silver nanowires, conductive polymers such as poly(3,4-ethylenedioxythiophene) (PEDOT), and carbon-based transparent conductive films such as carbon nanotubes and graphene. In a preferred embodiment, the transparent conductive film 2 can be used as an electrode in electrochemiluminescence immunoassay. In this case, the transparent conductive film 2 may be used as a working electrode or as a counter electrode.

Since the transparent conductive film 2 has transparency to visible light, when it is used for electrochemiluminescence immunoassay, a photodetector can be placed on the transparent conductive film side of the laminated body. In an embodiment in which the laminated body further comprises a transparent insulating substrate, a photodetector can also be placed on the transparent insulating substrate side of the laminated body. This enables detection of electrochemiluminescence near the emission interface, increases the light collection angle, and avoids reabsorption of the electrochemiluminescence signal by components in the solution, thereby allowing higher sensitivity.

When the laminated body is used as a biochip for electrochemiluminescence immunoassay, luminescence from electrochemiluminescent substances immobilized on the porous membrane can also be efficiently collected, enabling higher sensitivity. In the electrochemiluminescence process of a biochip comprising the laminated body, luminescence occurs when reactive species generated by the electrode reaction of the co-reactant reach the electrochemiluminescent substance before being deactivated. Thus, luminescence mainly arises from electrochemiluminescent substances immobilized in the porous membrane near the transparent conductive film. If a conductive film without light transmittance were used, it would be necessary to place the photodetector on the surface side of the porous membrane opposite to the conductive film, and luminescence generated near the electrode would be attenuated in the porous membrane before reaching the photodetector. In contrast, when using a laminated body comprising a transparent conductive film and a porous membrane, the distance from the luminescence site to the photodetector is short, allowing detection of electrochemiluminescence without significant attenuation.

In the present specification, the term "transparent conductive film" refers to a conductive film having a light transmittance of at least 50%. Preferably, the film has a light transmittance of at least 75%, and more preferably at least 90%. The light transmittance can be measured, for example, using a UV-visible spectrophotometer.

The transparent conductive film 2 preferably has low electrical resistance, for example, a sheet resistance of 2000 Ω/sq or less. If the sheet resistance exceeds 2000 Ω/sq, when current flows through the transparent conductive film during electrochemical measurement, the voltage drop generated in the transparent conductive film becomes large, thereby limiting the current and reducing the luminescence intensity, which is undesirable. In a more preferred embodiment, the sheet resistance of the transparent conductive film 2 is 1000 Ω/sq or less, and more preferably 500 Ω/sq or less. The sheet resistance is defined as the effective electrical resistance of the conductive film, calculated as resistivity divided by film thickness. By reducing the resistivity of the transparent conductive film and increasing its thickness, the sheet resistance can be adjusted to a preferred value. However, increasing the film thickness reduces the light transmittance, which may lead to a decrease in the intensity of electrochemiluminescence signals obtained in electrochemiluminescence measurement.

In a preferred embodiment, the transparent conductive film 2 is a transparent carbon-based conductive film, and in a more preferred embodiment, the transparent conductive film 2 is a graphene film.

The transparent carbon-based conductive film is a light-transmissive conductive film made of carbon materials such as graphene, graphite, glassy carbon, carbon nanotubes, or fullerenes. The transparent carbon-based conductive film also encompasses transparent conductive films produced by pyrolysis of polymeric materials such as photoresists, as disclosed in Non-Patent Document 2.

Methods for forming transparent carbon-based conductive films are known to those skilled in the art. A known method suitable for each type of carbon-based conductive film may be employed. For example, reference can be made to the following descriptions.

When a graphene film is used as the transparent carbon-based conductive film, the graphene film may be a monolayer or may consist of multiple layers. When the graphene film consists of multiple layers, it is preferable from the viewpoint of light transmittance that the film have two to three layers, although this is not limiting.

The graphene film may also be doped by substitution of carbon atoms with nitrogen, boron, or the like, or by molecular adsorption. In multilayer graphene films, metal chlorides or the like may be intercalated between the layers.

The graphene film preferably is a continuous film. A "continuous film" refers to a film having no holes or tears, in which the constituent components are continuously connected. When the graphene film is a continuous film, it has a uniform surface and a low sheet resistance, which is advantageous.

In the present specification, the term "monolayer graphene film" includes films that are substantially formed of a monolayer graphene film while partially including multilayer graphene film. A film "substantially formed of a monolayer graphene film" refers, for example, to one in which the multilayer graphene coverage area on the graphene film is 15% or less, 10% or less, 5% or less, or 3% or less. When the multilayer graphene coverage area on the graphene film is 3% or less, the monolayer graphene coverage in the graphene film is 97% or more.

Methods for forming graphene films are known to those skilled in the art (see, for example, Non-Patent Document 3). It is preferable to form the graphene film by chemical vapor deposition (CVD). However, as long as the laminated body 1 can function properly, graphene films formed by known methods such as mechanical exfoliation, chemical exfoliation, thermal decomposition of SiC, or reduction of graphene oxide may also be used.

The porous membrane 3 is laminated on the first surface of the transparent conductive film 2. The porous membrane 3 may be laminated so as to cover the entire surface of the transparent conductive film 2, or only a part of the surface. The porous membrane 3 may be one that is permeable to an electrolyte solution.

As used herein, "permeability to an electrolyte solution" means that a target compound for electrochemiluminescence detection, an electrochemiluminescent labeling compound, a co-reactant, cations such as sodium ions, anions such as chloride ions, and water molecules contained in the electrolyte solution can pass through the porous membrane 3 and reach the surface of, or the vicinity of, the transparent conductive film 2. By being permeable to the electrolyte solution, the electrochemiluminescent species and the co-reactant in the electrolyte solution can approach the vicinity of the surface of the transparent conductive film 2, so that, when a potential is applied to the transparent conductive film 2, electron transfer between those species and the transparent conductive film 2 becomes possible. Accordingly, the porous membrane 3 has pores continuous from one surface to the other, so that the electrochemiluminescent species and the co-reactant can permeate through the membrane.

Examples of the porous membrane 3 that can be used in the present invention include, but are not limited to, membranes made of materials selected from the group consisting of nitrocellulose, cellulose acetate, polyethersulfone, polytetrafluoroethylene, polyamide, polyvinylidene fluoride, regenerated cellulose, polycarbonate, polypropylene, polyvinylidene chloride, aluminum oxide, glass fibers, quartz fibers, polymethyl methacrylate, polystyrene, polyethylene, polyethylene terephthalate, and ceramics, or mixed membranes made of two or more of the materials selected from the group. Furthermore, the porous membrane 3 may be a laminated body of two or more porous membranes.

The structure and pore size of the porous membrane 3 can be optimized as appropriate. For example, it is preferable to optimize them in consideration of the combination with other constituent materials in the biochip of the present invention.

Although not limited thereto, the thickness of the porous membrane 3 is preferably 10 nm to 100 µm. If it is thinner than 10 nm, the thickness is smaller than the size of an antibody, and thus the benefit of increasing the amount of modification by utilizing the porous membrane to three-dimensionally enlarge the modification sites cannot be obtained. If it is thicker than 100 µm, in the electrochemiluminescence process, radical species generated by the electrode reaction of the co-reactant may become deactivated before reacting with electrochemiluminescent labeling substances immobilized in the porous membrane, resulting in decreased luminescence intensity, which is undesirable. In a preferred embodiment, the lower limit of the thickness of the porous membrane 3 is 100 nm or more, and more preferably 300 nm or more. In another preferred embodiment, the upper limit of the thickness of the porous membrane 3 is 50 µm or less, and more preferably 10 µm or less.

In an embodiment in which a graphene film produced by a CVD method is used as the transparent conductive film 2, the porous membrane 3 preferably supports the graphene film after the metal catalyst substrate has been removed by etching, in order to transfer the graphene film grown on the metal catalyst substrate onto a desired substrate. For the porous membrane 3 to function as such a support film, it is preferable that its thickness is 100 nm or more. If the thickness is less than 100 nm, the supporting strength of the graphene film formed by the CVD method becomes insufficient, and tearing is likely to occur during transfer. In addition, when transferring a graphene film or other carbon-based conductive film onto a hydrophilic substrate such as a glass substrate, it is preferable that the thickness of the porous membrane 3 be 200 µm or less. As the thickness increases, the internal stress of the porous membrane also increases, and if the thickness exceeds 200 µm, the transferred film may delaminate from the substrate.

Although not limited thereto, the average pore diameter of the porous membrane 3 is preferably 20 nm to 20 µm. If the diameter is less than 20 nm, immobilized antibodies or other components such as blocking agents may clog the pores of the porous membrane, thereby hindering the movement of components in the solution. If the diameter exceeds 20 µm, the density of the porous membrane decreases, its supporting strength during transfer of the graphene film becomes weak, and tearing of the film is likely to occur, which is undesirable. In a preferred embodiment, the average pore diameter of the porous membrane 3 is 200 nm to 10 µm. As used herein, the average pore diameter of the porous membrane 3 refers to the mean value of the diameters of pores present on the surface of the porous membrane. The average pore diameter is determined by observing the surface of the porous membrane with a scanning electron microscope (SEM) or the like, randomly selecting several hundred pores (for example, 300 pores), measuring the area of each pore, calculating the diameter of each pore assuming the pore is circular, and averaging these values to obtain the surface average pore diameter.

Although not limited thereto, the porous membrane 3 preferably has a pore area ratio of 20% or more relative to the membrane surface. If the pore area ratio relative to the membrane surface is less than 20%, the electrolyte solution permeability of the porous membrane 3 decreases, which is undesirable. The upper limit of the pore area ratio relative to the membrane surface is not particularly limited as long as the porous membrane 3 functions as a transfer support film for the transparent conductive film 2, but it may be, for example, 60% or less. In a preferred embodiment, the pore area ratio relative to the membrane surface is 25% or more, and in a more preferred embodiment is 30% or more. The pore area ratio relative to the membrane surface of the porous membrane 3 means the ratio of the total area of all pores present on one surface of the porous membrane to the geometric area of that surface. The pore area can be measured by observing the surface of the porous membrane using SEM or the like.

In one embodiment, the porous membrane 3 is light-transmissive at the emission wavelength of the electrochemiluminescent substance. In particular, it is preferable that the porous membrane 3 does not absorb the luminescence generated by electrochemiluminescence. The term "light-transmissive," as used herein with respect to the porous membrane 3 means that the membrane has a light transmittance of 50% or more. Preferably, the membrane has a light transmittance of 75% or more, and more preferably 90% or more. The light transmittance can be measured, for example, using a UV-visible spectrophotometer.

In one embodiment, the porous membrane 3 has protein adsorption capability. The term "protein adsorption capability," as used herein, refers to the ability of the porous membrane to immobilize proteins such as antibodies for electrochemiluminescence immunoassay in a manner suitable for measurement. The protein adsorption capability can be evaluated by quantifying the amount of protein in a solution before and after adsorption to the porous membrane 3 using, for example, Bradford reagent. By having protein adsorption capability, the porous membrane 3 allows easy immobilization of proteins necessary for electrochemiluminescence immunoassay. The term "protein," as used herein, is not limited as long as it can be used for immunoassay. Examples include antibodies, fragments or variants thereof, lectins, and portions of proteins (domains, polypeptides), but the invention is not limited thereto.

In one embodiment, the porous membrane 3 may include immobilized compounds. In the present invention, the compounds immobilized on the porous membrane 3 are substances that specifically bind to the target compound to be detected. Such specific binding substances are not limited to proteins as long as they can be immobilized on the porous membrane 3 and can specifically immobilize the target compound within the porous membrane through specific interactions. Examples of such specific binding substances include proteins, peptides, amino acids, lipids, saccharides, DNA, RNA, receptors, and haptens. No particular limitation is imposed on molecular weight or whether they are of natural or synthetic origin. For example, antibodies or antigens that can be used in immunological assay methods utilizing immune reactions are suitably employed. Other specific binding substances may include low-molecular compounds, high-molecular compounds, organic compounds, inorganic compounds, polysaccharides, polymers, lectins, and compounds composed of combinations thereof. The method for immobilizing these compounds on the porous membrane 3 is not particularly limited as long as electrochemiluminescence immunoassay can be performed, and known methods such as physical adsorption or chemical bonding can be employed.

In the present specification, the term "electrochemiluminescence immunoassay" is not limited to measurement of electrochemiluminescence utilizing antigen-antibody reactions, but also encompasses measurement of electrochemiluminescence utilizing binding between compounds such as those described above.

In one embodiment, the laminated body of the present invention further comprised a transparent insulating substrate, with the porous membrane laminated on the first surface of the transparent conductive film and the transparent insulating substrate laminated on the second surface of the transparent conductive film. An embodiment of such a laminated body is shown in Fig. 2. As shown in Fig. 2, the laminated body 1' is composed of a transparent conductive film 2, a porous membrane 3, and a transparent insulating substrate 4.

The transparent insulating substrate 4 is not limited as long as it is a substrate capable of transmitting light from electrochemiluminescence generated on the transparent conductive film 2, and known transparent insulating substrates can be used. By using a transparent insulating substrate, the light generated by electrochemiluminescence can be collected by a photodetector placed on the transparent insulating substrate 4 side of the laminated body 1. Placing the photodetector on the transparent insulating substrate 4 side of the laminated body 1 shortens the distance from the luminescence site to the photodetector, thereby improving light-collection efficiency.

Examples of transparent insulating substrates usable in the present invention include, but are not limited to, quartz, glass, other transparent glass materials, polyethylene terephthalate, polyester, acrylic resin, polyethylene, nylon, polyvinyl chloride, and polyimide.

In one embodiment, the transparent insulating substrate 4 may further comprise other components used for electrochemiluminescence measurement, such as a contact electrode for electrical connection or a conductive additive. The contact electrode may be any that can directly contact the transparent conductive film and allow current flow, and its form and material are not limited. For example, a contact electrode made of chromium or gold may be used.

Known conductive additives can also be used. Examples of transparent conductive additives include poly(3,4-ethylenedioxythiophene): polystyrene sulfonate (PEDOT:PSS). When the transparent insulating substrate 4 is coated with a conductive additive, the porous membrane/transparent conductive film laminated body is laminated on the surface of the transparent insulating substrate 4 coated with the conductive additive.

In one embodiment, the laminated body of the present invention can be used as an electrode for electrochemical measurement. When used for electrochemical measurement, the laminated body may be employed as a working electrode or counter electrode in a two-electrode system consisting of a working electrode and a counter electrode, a three-electrode system consisting of a working electrode, a counter electrode, and a reference electrode, or an electrode system having four or more electrodes. In a preferred embodiment, the laminated body is used as a working electrode.

When the laminated body of the present invention is used as an electrode for electrochemical measurement, since the porous membrane is formed on the solution side, it has excellent durability compared with electrodes such as graphene electrodes without a laminated porous membrane, being less likely to delaminate from the substrate or tear. Depending on the measurement conditions, the laminated body of the present invention can be used repeatedly as an electrode for electrochemical measurement (two, three, four, five, six, seven, eight, nine, ten times, or more).

Another aspect of the present invention provides a biochip comprising a laminated body in which a porous membrane, a transparent conductive film, and a transparent insulating substrate are laminated in this order. The biochip according to the present invention is preferably a biochip for electrochemical measurement, and more preferably a biochip for electrochemiluminescence immunoassay.

A schematic diagram of an embodiment of the biochip according to the present invention is shown in Fig. 3. As shown in Fig. 3, the biochip 11 comprises a laminated body in which a porous membrane 3, a transparent conductive film 2, and a transparent insulating substrate 4 are laminated. A specific binding substance for a target compound to be detected is immobilized on the porous membrane 3. In addition, between the transparent conductive film 2 and the transparent insulating substrate 4, a contact electrode 5 for electrical connection is partially laminated. The biochip 11 further comprises a membrane 6 on the surface of the porous membrane 3 of the laminated body 1, and on the surface of the membrane 6, an electrical conductor 7, a conjugate pad 8, and an absorbent pad 10 are provided. The conjugate pad 8 further comprises a sample pad 9. The biochip 11 having such a configuration enables electrochemiluminescence immunoassay when the biochip is placed with the transparent insulating substrate 4 side facing a photodetector 12. The schematic diagram shown in Fig. 3 merely illustrates one embodiment, and the biochip of the present invention is not limited to this configuration as long as it can be used for electrochemiluminescence immunoassay.

When a sample in which the target compound and a co-reactant are pre-mixed is supplied to the sample pad 9 of such a biochip, the target compound passes through the sample pad 9 and flows downstream to the conjugate pad 8. In the conjugate pad 8, the target compound comes into contact with an electrochemiluminescent substance-labeled compound, forming a complex as they pass through the pad. The complex and the co-reactant are then developed onto the membrane 6, which is disposed in contact with the lower surface of the conjugate pad. The complex and co-reactant developed on the membrane 6 infiltrate into the porous membrane 3 of the laminated body 1. Since a specific binding substance that binds to the target compound is immobilized in part of the porous membrane 3, the complex binds and is immobilized there. Because the immobilized complex is bound to an electrochemiluminescent substance-labeled compound, when a voltage is applied between the transparent conductive film 2 and the electrical conductor 7, an electrode reaction of the co-reactant occurs, leading to electrochemiluminescence resulting from the reaction between the electrode reaction products and the electrochemiluminescent substance. The target compound is detected by measuring the luminescence with the photodetector 12.

In one embodiment, the biochip of the present invention includes a laminated body comprising a transparent conductive film, a porous membrane, and a transparent insulating substrate, and a membrane. In another embodiment, the biochip of the present invention includes a laminated body comprising a transparent conductive film, a porous membrane, and a transparent insulating substrate, a membrane, and an electrical conductor. In another embodiment, the biochip of the present invention includes a laminated body comprising a transparent conductive film, a porous membrane, and a transparent insulating substrate, a membrane, an electrical conductor, and a conjugate pad. In another embodiment, the biochip of the present invention includes a laminated body comprising a transparent conductive film, a porous membrane, and a transparent insulating substrate, a membrane, an electrical conductor, a conjugate pad, and a sample pad. In addition, the biochip may include a configuration used in lateral-flow immunochromatographic detection methods employing known test strips such as nitrocellulose membranes. Such configurations are disclosed, for example, in Japanese Patent Publication No. 6464308. In the biochip, as long as one of the laminated bodies and the additional electrical conductor functions as a working electrode and the other as a counter electrode, either may serve as the working electrode.

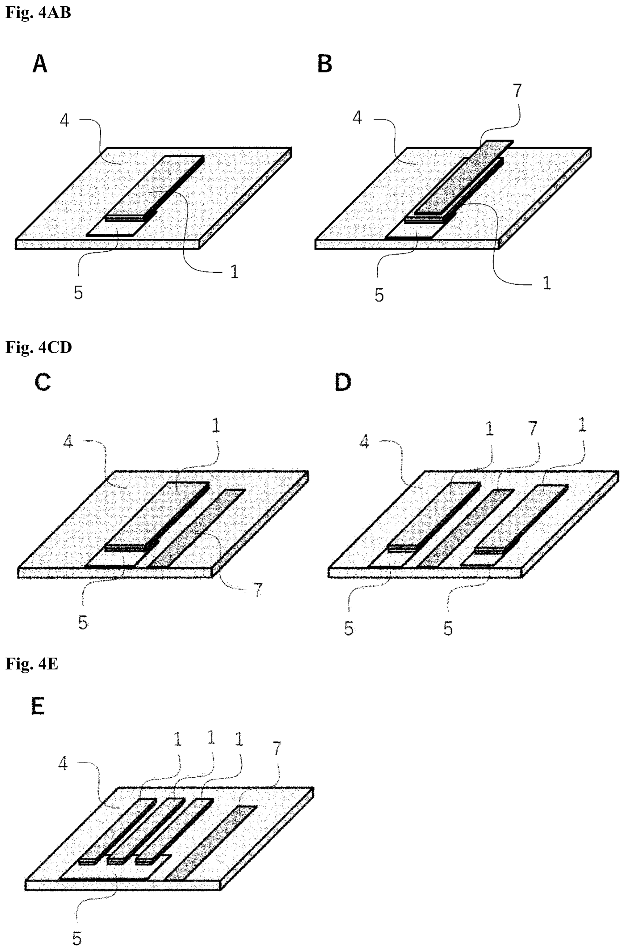

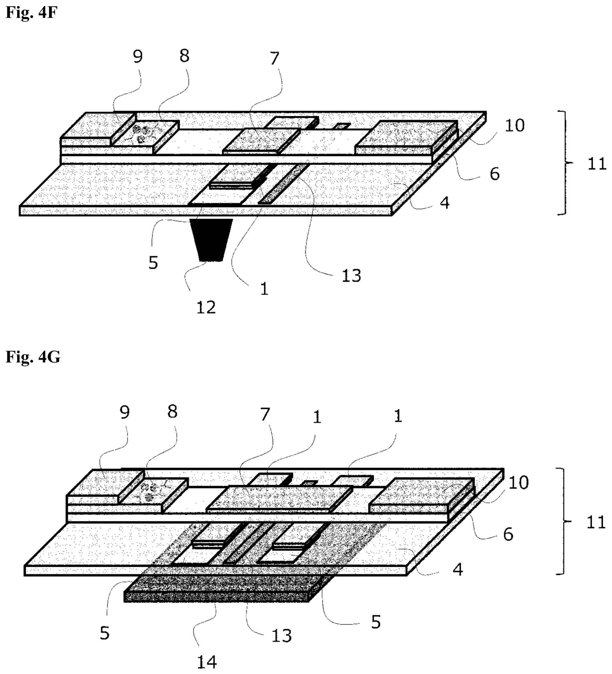

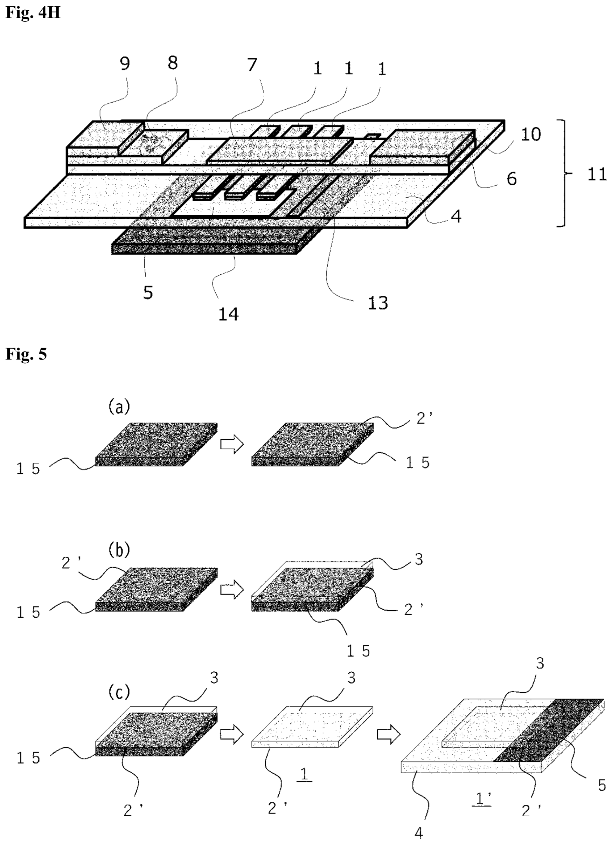

Examples of embodiments of the biochip are illustrated in Figs. 4A-4F. Fig. 4A shows a biochip having a contact electrode 5 between the laminated body 1 and the transparent insulating substrate 4. Fig. 4B shows a biochip in which the laminated body 1 and the electrical conductor 7 are sequentially laminated on the transparent insulating substrate 4, with a contact electrode 5 provided between the laminated body 1 and the transparent insulating substrate 4. Fig. 4C shows a biochip in which the laminated body 1 and the electrical conductor 7 are independently laminated on the same surface of the transparent insulating substrate 4, with a contact electrode 5 provided between the laminated body 1 and the transparent insulating substrate 4. Fig. 4D shows a biochip in which two laminated bodies 1 and the electrical conductor 7 are independently laminated on the same surface of the transparent insulating substrate 4, with a contact electrode 5 provided between the laminated body 1 and the transparent insulating substrate 4. Fig. 4E shows a biochip having three laminated bodies 1 and one electrical conductor 7 independently provided on the same surface of the transparent insulating substrate 4, with a single contact electrode 5 between the laminated bodies 1 and the transparent insulating substrate 4. Fig. 4F shows a biochip further comprising a reference electrode 13 on the transparent insulating substrate of the biochip shown in Fig. 3. Figs. 4G and 4H show biochips, based on the biochip shown in Fig. 4F, including multiple laminated bodies 1. Figs. 4G and 4H also illustrate embodiments in which electrochemiluminescence is measured with an image sensor 14 instead of the photodetector 12.

In the embodiments shown in Figs. 4C-4E, the two-electrode system consists of the transparent conductive film 2 and the electrical conductor 7, which are brought into contact via a membrane infiltrated with an electrolyte solution.

As the membrane 6, any material may be used as long as it allows penetration and development of the target compound, electrochemiluminescent substance-labeled reagent, and co-reactant. Examples include porous membranes of polyethylene, polyethylene terephthalate, nylon, glass, polysaccharides, including cellulose and cellulose derivatives such as nitrocellulose, and ceramics, but the invention is not limited thereto. In a preferred embodiment, the membrane 6 does not exhibit absorption at the emission wavelength of electrochemiluminescence. In a more preferred embodiment, the membrane 6 has light transmittance at the emission wavelength of electrochemiluminescence.

The electrical conductor 7 is used as a counter electrode or a working electrode in a two-electrode system, a three-electrode system, or an electrode system having four or more electrodes for electrochemical measurement. As the electrical conductor 7, known electrodes used for electrochemiluminescence measurement can be employed. Examples include platinum, gold, silver, copper, stainless steel, nickel, aluminum, and carbon electrodes. The electrical conductor 7 can be fixed onto the membrane 6 by an adhesive or a pressing member. In a preferred embodiment, the electrical conductor 7 functions as a reflector. By reflecting photons emitted in a direction opposite to the transparent insulating substrate among the electrochemiluminescence generated near the surface of the transparent conductive film 2 of the biochip, the electrical conductor 7 improves the light-collection efficiency of the photodetector. Examples of preferred materials for the electrical conductor 7 include, but are not limited to, platinum, gold, silver, copper, stainless steel, nickel, aluminum, and graphene films formed on metallic substrates of these metals.

The conjugate pad 8 contains an electrochemiluminescent substance-labeled reagent that specifically reacts with the target compound. The method of producing a conjugate pad containing such a reagent is not limited, and for example, it can be produced by impregnating the conjugate pad with the reagent and then drying it. Examples of suitable materials for the conjugate pad include paper, cellulose mixtures, nitrocellulose, polyester, acrylonitrile copolymers, and nonwoven fibers such as glass fibers and rayon, but the invention is not limited thereto.

The sample pad 9 serves as a sample supply section for receiving a sample, and includes any substance and form that, in a pad-shaped state, can absorb a liquid sample and allow passage of the liquid and components of the target compound. Examples of materials for the sample pad 9 include glass fibers, acrylic fibers, hydrophilic polyethylene materials, dried paper, paper pulp, and textiles, but the invention is not limited thereto. The sample pad 9 may also serve the function of the conjugate pad 8. The sample pad 9 may additionally contain suitable reagents such as erythrocyte agglutination agents or blocking reagents.

The configuration and form of the membrane 6, the conjugate pad 8, the sample pad 9, and the absorbent pad 10, the materials of each component, and the methods for laminating and retaining the components can be those used in known immunochromatographic methods.

In addition to the above configuration, the biochip of the present invention may further comprise additional pads disposed between the sample pad 9 or the conjugate pad 8 and the porous membrane for removing components unnecessary for detection of the target compound, reference electrodes, reflectors, and the like.

In the electrochemiluminescence immunoassay using the laminated body or biochip of the present invention, the target compound can be captured in the porous membrane by a specific binding substance. The type of target compound is not particularly limited as long as it is detectable by electrochemiluminescence immunoassay. Examples of such target compounds include low-molecular-weight compounds, macromolecular compounds, organic compounds, inorganic compounds, polysaccharides, polymers, lipids, nucleic acids and their analogs, polypeptides, proteins, antibodies, lectins, and compounds composed of combinations thereof. In a preferred embodiment, the target compound is one that is present or potentially present in a biological sample. Examples of such target compounds suitably include microorganisms such as bacteria and viruses, and disease-related markers such as cancer markers. More specific examples include proteins, polypeptides, amino acids, nucleic acids (DNA, RNA), and sugar chains contained in biological samples.

In the present specification, the term "biological sample" refers to, for example, tissues, cells, body fluids, or peritoneal lavage fluids. The term "body fluid" as used herein means a liquid biological sample collected from a living body. Examples include blood (including serum, plasma, and interstitial fluid), cerebrospinal fluid, urine, fecal matter, saliva, lymph fluid, digestive fluids, ascites, pleural effusion, perineural root fluid, and extracts of tissues or cells. Blood is preferred. A biological sample that has been subjected to pretreatment such as purification or concentration according to the compound derived from the biological sample may also be used. Purification or concentration of a biological sample can be carried out using known methods as appropriate depending on the target compound to be detected.

Another aspect of the present invention provides a kit for electrochemiluminescence immunoassay comprising the above biochip. The kit comprises at least the biochip and may further comprise reagents necessary for measurement (for example, a reagent containing an electrochemiluminescent substance, a reagent containing a co-reactant, and an electrolyte solution), a diluent for specimens, test tubes, instruments for collecting, storing, or transporting biological samples, instruction manuals, and the like.

Another aspect of the present invention provides a method for detecting a target compound by electrochemiluminescence immunoassay using the laminated body or the biochip of the present invention described above. The detection method comprises the following steps:
(a) binding the target compound with an electrochemiluminescent substance;
(b) capturing the target compound in the porous membrane of the laminated body, wherein the target compound is captured by a specific binding substance for the target compound that is immobilized in the porous membrane; and
(c) after steps (a) and (b), applying a potential to the laminated body to measure electrochemiluminescence.

In the detection method according to the present invention, either step (a), binding the target compound with the electrochemiluminescent substance, or step (b), capturing the target compound in the porous membrane of the laminated body, may be performed first. That is, in the detection method of the present invention, step (a) may be followed by step (b), or step (b) may be followed by step (a), and both embodiments are encompassed.

Electrochemiluminescence immunoassay is a method in which a target compound is captured on or near a working electrode through an antigen-antibody or other specific binding reaction, and electrochemiluminescence generated by binding the captured compound to an electrochemiluminescent substance is used to detect the target compound. The laminated body used in the detection method of the present invention comprises a transparent conductive film, a porous membrane, and a transparent insulating substrate, and since it does not contain rare elements, it can be manufactured at low cost and used as a disposable electrode. Accordingly, it is suitable for application in rapid and simple tests and POCT devices.

Electrochemiluminescent substances that can be used in electrochemiluminescence immunoassay employing the laminated body or biochip of the present invention may be those known in the art. Electrochemiluminescent substances or their co-reactants may be selected from compounds disclosed, for example, in S. Parveen, M. S. Aslam, L. Hu, and G. Xu, Electrogenerated Chemiluminescence: Protocols and Applications, Heidelberg, 2013, pp. 18-31 and pp. 44-60; Z. Liu et al., "Recent advances in electrochemiluminescence," Chemical Society Reviews, 44, pp. 3117-3142 (2015), https://doi.org/10.1039/CSCS00086F; and Japanese Patent Publication No. 3574457. Examples include, but are not limited to, a combination of tris(2,2'-bipyridyl)ruthenium(II) and tripropylamine as the co-reactant; a combination of tris(2,2'-bipyridyl)ruthenium(II) and 2-(di-n-butylamino)ethanol as the co-reactant; and a combination of luminol and hydrogen peroxide as the co-reactant.

The detection method according to the present invention includes step (a) of binding the target compound with an electrochemiluminescent substance.

The electrochemiluminescent substance binds to the target compound through a means that specifically binds to the target compound. Such a means preferably includes an antibody, but is not limited thereto. In other words, the electrochemiluminescent substance includes an electrochemiluminescent labeling compound. Examples of the electrochemiluminescent substance-labeled compound include, for instance, antibodies conjugated with compounds such as tris(2,2'-bipyridyl)ruthenium(II).

To bind the target compound with the electrochemiluminescent substance, the two compounds may simply be mixed in a solution. The solution can be appropriately selected depending on the target compound and the electrochemiluminescent substance to be used. Examples include, but are not limited to, commonly used buffers such as phosphate buffer, Tris buffer, and Good's buffer. The pH of the buffer is preferably in the range of 6.0 to 9.5 and may be appropriately adjusted according to the nature of the target compound and the electrochemiluminescent substance to be used. The mixing conditions may be, for example, gently mixing at 37°C for 30 minutes using a shaker.

When step (a) is carried out after step (b), a solution containing the electrochemiluminescent substance may be added into the porous membrane so that the target compound captured in the porous membrane binds to the electrochemiluminescent substance. In this case, any unbound electrochemiluminescent substance may be washed away with a buffer or the like, if necessary.

The detection method according to the present invention includes step (b) of capturing the target compound in the porous membrane of the laminated body, wherein the target compound is captured by a specific binding substance for the target compound that is immobilized in the porous membrane.

To capture the target compound in the porous membrane, a specific binding substance for the target compound is immobilized in the porous membrane in advance. The method for immobilizing the specific binding substance on the porous membrane is not limited, and known methods for immobilizing compounds on porous membranes, such as physical adsorption and chemical bonding, can be employed. For example, a solution containing a desired protein (such as an antibody against an antigen) may be adjusted to a predetermined concentration (e.g., 0.1-5 mg/mL), and a fixed amount of this solution may be applied to the porous membrane. In this way, the protein can be immobilized on the porous membrane.

The solution used to apply the specific binding substance to the porous membrane is not limited but may preferably be a commonly used buffer such as phosphate buffer, Tris buffer, or Good's buffer. The pH of the buffer is preferably in the range of 6.0 to 9.5 and may be appropriately set according to the properties of the specific binding substance. The buffer may further contain salts such as NaCl, stabilizers or storage agents such as sucrose, and antiseptics such as ProClin. Salts include those added to adjust ionic strength, such as NaCl, as well as those added during adjustment of buffer pH, such as sodium hydroxide. After immobilizing the specific binding substance on the porous membrane, blocking may also be performed by covering the immobilized region or other parts with a commonly used blocking agent in solution or vapor form.

As described above, a solution containing the target compound is allowed to infiltrate into the porous membrane in which the specific binding substance has been immobilized. This enables capture of the target compound within the porous membrane. In one embodiment, after the capture step of the target compound in the porous membrane, a washing step is included to wash away unbound target compound.

As the solution for infiltrating the target compound into the porous membrane and as the washing solution, a solution of the same type as that used for applying the specific binding substance to the porous membrane can be employed. Alternatively, the electrolyte solution used for electrochemiluminescence immunoassay may also be used.

Examples of electrolyte solutions usable in electrochemiluminescence immunoassay employing the laminated body of the present invention as an electrode include, but are not limited to, commonly used buffers such as phosphate buffer, Tris buffer, and Good's buffer solution. The electrolyte solution preferably contains a co-reactant. The concentration of the co-reactant is not particularly limited and may be, for example, 0.01-0.3 mol/L. The electrolyte solution may further contain other components such as surfactants. Examples of surfactants include, but are not limited to, Tween 20, Tween 80, and Triton X-100. The concentration of the surfactant is not particularly limited and may be, for example, 0.01-2 wt%.

The detection method according to the present invention includes step (c) of applying a potential to the laminated body after steps (a) and (b) to measure electrochemiluminescence.

Through steps (a) and (b), the target compound is captured in the porous membrane of the laminated body, and the electrochemiluminescent substance is bound to the target compound. By immersing the laminated body in an electrolyte solution containing a co-reactant and applying a potential, the electrochemiluminescent substance in the porous membrane emits electrochemiluminescence. By measuring the resulting electrochemiluminescence, the target compound can be detected qualitatively or quantitatively. At this time, an additional electrode is used in contact with the electrolyte solution. The position of the additional electrode is not limited as long as current flows between the additional electrode and the transparent conductive film. Either the additional electrode or the transparent conductive film may serve as the working electrode.

The application of a potential to the working electrode is carried out using a voltage source such as a potentiostat in accordance with the potential at which an electrode reaction of the co-reactant contained in the electrolyte solution occurs. For example, when an electrolyte solution containing tripropylamine (TPrA) as a co-reactant is used, the potential of the working electrode is swept from 0 V to 1.6 V versus a silver/silver chloride reference electrode. At around 0.8 V and at higher potential ranges, TPrA is oxidized on the working electrode to generate tripropylamine radical cations (TPrAH⁺) near the electrode surface. A portion of the generated TPrAH⁺ releases a hydrogen ion (H⁺) in the electrolyte solution and is converted into a tripropylamine radical (TPrA·). When TPrA· migrates through the electrolyte solution and collides with, or reaches near, the electrochemiluminescent substance tris(2,2'-bipyridyl)ruthenium(II) (Ru(bpy)₃²⁺) immobilized in the porous membrane via the target compound and specific binding substance, Ru(bpy)₃²⁺ is reduced by TPrA· to form Ru(bpy)₃⁺. Furthermore, when TPrAH⁺ that has not released H⁺ collides with, or reaches near, Ru(bpy)₃⁺, Ru(bpy)₃⁺ is oxidized, generating the excited state Ru(bpy)₃²⁺*, which emits light upon returning to the ground state Ru(bpy)₃²⁺. The luminescence intensity can be measured using commercially available photodiodes or photomultiplier tubes. Photomultiplier tubes enable more sensitive measurement than photodiodes because they can detect weaker light. Imaging devices such as CMOS image sensors can also be used. By using an imaging device, the distribution of luminescence on the working electrode can be obtained as an image, making it possible to impart functions of test lines and control lines in immunochromatography or to perform multiplex analysis by pre-applying different types of specific binding substances to different positions in the porous membrane.

Another aspect of the present invention provides a method of using the above laminated body as a transparent electrode. The method of using the laminated body as a transparent electrode is characterized in that a solvent having the same refractive index as that of the porous membrane is employed.

According to this method, for example, during ECL immunoassay, electrochemiluminescence (ECL) can be detected by a photodetector without scattering within the porous membrane. This makes it possible to sharpen the boundary between luminescent and non-luminescent regions. When considering associating multiple analytes with luminescent positions of the analytical device, this method contributes to the integration of test items.

Those skilled in the art can adjust the refractive index of the solvent used when employing the laminated body as an electrode so that it matches the refractive index of the porous membrane. Such a solvent may be obtained, for example, by combining solvents having different refractive indices, such as glycerol and water, and adjusting the composition ratio so as to match the refractive index of the porous membrane. The solvent to be used is not particularly limited as long as it does not interfere with the use of the laminated body as an electrode.

Another aspect of the present invention provides a laminated body that can be used as an electrochromic device. The laminated body comprises, in sequence, a transparent insulating substrate, a transparent conductive film, a porous membrane, a transparent conductive film, and a transparent insulating substrate. In this case, by adding a solvent having the same refractive index as that of the porous membrane to the porous membrane, the laminated body can function as an electrochromic device in which the two transparent conductive films are used as a working electrode and a counter electrode.

Such a laminated body may further comprise an electrical conductor in contact with the transparent conductive film. In addition, the graphene film in the laminated body may be electrodeposited with materials such as Prussian blue or silver.

Another aspect of the present invention provides a method for producing a laminated body in which a transparent insulating substrate, a carbon-based conductive film, and a porous membrane are laminated in this order. The method for producing the laminated body comprises the following steps:
(a) forming a carbon-based conductive film on a metal catalyst substrate by a chemical vapor deposition (CVD) method;
(b) further forming a porous membrane on the carbon-based conductive film to produce a laminated body; and
(c) removing the metal catalyst substrate by etching and transferring the laminated body so that the carbon-based conductive film comes into contact with a transparent insulating substrate.

A scheme of the production method according to the present invention is shown in Fig. 5.

Conventionally, in producing an electrode having a graphene film as a carbon-based conductive film, a PMMA film was formed on the graphene film formed on a metal catalyst substrate as a transfer support film, and the process included removing the PMMA film after etching of the metal catalyst substrate and transferring the graphene film to a desired substrate. However, according to the production method of the present invention comprising the steps described above, a porous membrane is formed in place of the transfer support film, thereby also serving the role of the transfer support film. As a result, the step of removing the transfer support film is not required, making it possible to reduce labor, time, and production cost.

In addition, in conventional graphene film transfer methods using PMMA as a transfer support film, PMMA residues remain on the graphene film surface after PMMA removal. Such residues adversely affect the properties of the graphene film. In contrast, since the production method of the present invention does not include a step of removing a transfer support film, the above problem can be avoided.

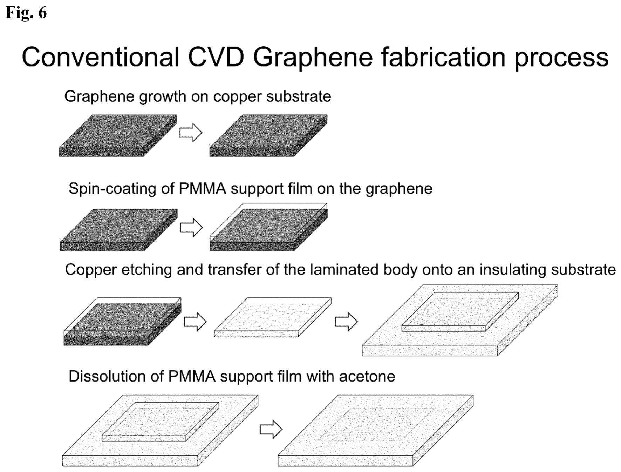

A conventional scheme for producing a graphene electrode using a transfer support film is shown in Fig. 6.

The method for producing a laminated body according to the present invention includes step (a) of forming a graphene film on a metal catalyst substrate by a chemical vapor deposition (CVD) method. Methods for forming a graphene film 2' on a metal catalyst substrate 15 by CVD are known, and may be carried out with reference to, for example, Non-Patent Document 3. The metal catalyst substrate 15 that can be used in the present invention is not limited, and known substrates used for the formation of graphene films 2' may be employed. Preferred examples include copper substrates, nickel substrates, cobalt substrates, iridium substrates, platinum substrates, gold substrates, and alloy substrates thereof. Substrates in which these metals are deposited on heat-resistant substrates may also be used.

After forming a graphene film on a metal catalyst substrate in step (a), step (b) of further forming a porous membrane on the graphene film is carried out.

The method for forming the porous membrane 3 on the graphene film is not limited, and known techniques such as spin coating, spray coating, coating methods using applicators or bar coaters, or vapor deposition may be used. In the following, a method for forming a porous membrane by spin coating will be described.

A polymer serving as a material for the porous membrane 3 is dissolved in a solvent to prepare a porous-membrane-forming solution. The polymer may be appropriately selected depending on the porous membrane 3 to be formed. One polymer may be used for forming the porous membrane 3, or two or more polymers may be used.

When forming the porous membrane 3 by spin coating, it is known that the film thickness and average pore diameter of the porous membrane vary depending on conditions such as the choice of solvent, the polymer concentration in the solvent, the rotation speed of the spin coater, the viscosity of the solution, and the average molecular weight of the polymer (which affects viscosity). Those skilled in the art can appropriately set the above conditions according to the polymer and solvent used so as to form a porous membrane having a desired film thickness and average pore diameter. Examples of conditions for forming the porous membrane 3 by spin coating are given below by way of illustration, but the invention is not limited thereto.

The solvent is not particularly limited as long as it can dissolve the polymer and form a porous membrane by spin coating. Examples of the solvent include acetone, methanol, diethyl ether, and ethyl acetate. Preferably, the solvent is a low-boiling solvent. In a preferred embodiment, the solvent has a boiling point of 100°C or lower, and in a more preferred embodiment, the solvent has a boiling point of 80°C or lower. Generally, low-boiling solvents have fast evaporation rates, so using a low-boiling solvent increases the precipitation rate of the polymer in the solution, thereby promoting random precipitation of the polymer and formation of a porous structure, which is preferred.

In one embodiment, it is preferable that the solvent be a mixed solvent of a good solvent and a poor solvent for the polymer. In a mixed solvent of a good solvent and a poor solvent, during evaporation of the solvent, as the good solvent decreases, the mixed solvent gradually changes into a poorer solvent, resulting in phase separation. This phase separation promotes formation of porosity in the polymer. Those skilled in the art can appropriately select good and poor solvents for the polymer. For example, when the polymer is cellulose acetate, an acetone/formamide mixed solvent may be used. Other examples of mixed solvents include acetone/dimethyl sulfoxide, but the invention is not limited thereto. The mixing ratio of good solvent to poor solvent is not particularly limited as long as porosity is promoted; for example, the volume ratio of good solvent to poor solvent may be 99:1 to 1:1.

The polymer concentration in the solvent is not particularly limited but may be, for example, 0.5-20 wt%.

A laminated body of the metal catalyst substrate 15 and graphene film 2' is fixed on the stage of a spin coater, and the prepared porous-membrane-forming solution is dropped onto the surface of the graphene film 2', particularly near the center. The amount of the porous-membrane-forming solution to be dropped only needs to be sufficient to cover the graphene film 2'. The rotation speed and rotation time of the spin coater are not particularly limited as long as a desired porous membrane can be formed. For example, the rotation speed may be 200-5000 rpm, and the rotation time may be 10-120 seconds. Thereafter, if the solvent remains, drying by heating is performed.

In a preferred embodiment, the porous membrane 3 may be cast using an applicator or the like and formed on the graphene film by a phase inversion method.

The phase inversion method is a method in which materials such as polymers undergo pore formation during phase transition from the liquid state to the solid state under controlled conditions. Representative phase inversion methods include nonsolvent-induced phase separation and thermally induced phase separation. In nonsolvent-induced phase separation, one phenomenon occurs when the evaporation of a solvent in a polymer solution containing a nonsolvent or a poor solvent destabilizes the polymer solution and the polymer precipitates in the presence of the nonsolvent. Another phenomenon occurs when a nonsolvent comes into contact with the polymer solution, extracting the solvent into the nonsolvent and at the same time penetrating into the polymer solution, thereby destabilizing the polymer solution and causing precipitation of the polymer. The former is referred to as the dry phase inversion method, and the latter as the wet phase inversion method. A combination of both is referred to as the dry-wet phase inversion method. Methods for forming porous membranes by phase inversion are known, and reference may be made to, for example, Masahiro Tamura, Tadashi Uragami, Mizuho Sugihara, "Permeation Characteristics on Cellulose Nitrate-Cellulose Acetate Blend Polymer Membranes," Journal of the Japan Society of Colour Material, Vol. 50 (6), 317-322 (1977).

The solvents used for the phase inversion method may be the same as those used for spin coating. The polymer concentration in the solvent may be 0.5-20 wt%.

After forming the porous membrane on the graphene film in step (b), step (c), removing the metal catalyst substrate by etching is carried out. Known methods can be employed to remove the metal catalyst substrate 15. For example, when a copper foil is used as the metal catalyst substrate 15, it can be etched with an aqueous solution of iron nitrate (Fe(NO₃)₃). If graphene is also formed on the backside of the metal catalyst substrate 15, where the porous membrane 3 has not been formed, the backside graphene film is removed prior to etching the metal catalyst substrate 15 by oxygen plasma treatment or the like. The porous membrane 3/graphene film 2'/metal catalyst substrate 15 is floated on the etching solution so that the side from which the graphene has been removed comes into contact with the etching solution, thereby removing the metal catalyst substrate 15. After removal of the metal catalyst substrate 15, the laminated body composed of the porous membrane 3 and the graphene film 2' is transferred to the water surface of a bath of pure water for washing. The washing step is preferably performed three times or more. The laminated body 1 floating on the pure water is then scooped up with a desired transparent insulating substrate 4. After scooping, the laminated body 1 is dried to complete the transfer of the graphene. Prior to scooping, it is preferable to pre-form a contact electrode 5 for electrical conduction to the graphene on the transparent insulating substrate 4. The scooping is performed so that a part of the graphene film 2' is placed on the contact electrode 5. In this manner, a laminated body 1' composed of the graphene film 2', the porous membrane 3, and the transparent insulating substrate 4 can be produced.

Hereinafter, the present invention will be described in more detail by way of examples; however, the invention is not limited to the embodiments described below.

### (Example 1: Production of a laminated body comprising a graphene film and a porous membrane)

In this example, a laminated body composed of a monolayer graphene film and a cellulose membrane (porous membrane) (hereinafter also referred to as "porous membrane/graphene laminated body") was produced as follows.

Copper foil (35 µm thick) was prepared as the metal catalyst substrate for the growth of graphene by chemical vapor deposition (CVD). Prior to graphene film formation by CVD, the copper foil was sequentially immersed in pure water, ethanol, and acetone, followed by ultrasonic treatment to clean the surface of the copper foil. Thereafter, the copper foil was transferred into a thermal CVD furnace, hydrogen was introduced, and hydrogen annealing of the copper substrate was carried out at 1000°C under a hydrogen atmosphere at a total pressure of 700 Pa for 30 minutes. Subsequently, methane was introduced, and CVD growth of graphene was carried out for 10 minutes at 1000°C and a total pressure of 750 Pa, with flow rates of 2 sccm methane and 20 sccm hydrogen.

Using a spin coating method, a porous membrane was formed on the graphene film grown on the copper foil.

As the support layer for graphene transfer, nitrocellulose (CN), cellulose acetate (CA), or a mixed membrane of nitrocellulose and cellulose acetate (mixed cellulose ester membrane, MCE) was used. For preparation of the porous membrane-forming solution, nitrocellulose (Nacalai Tesque, product No. 24728-34), cellulose acetate (Sigma-Aldrich, product No. 419028), and acetone as a solvent were prepared. A solution was prepared by mixing cellulose acetate or nitrocellulose with acetone so that the content of cellulose acetate or nitrocellulose became 2 wt%. Further, three types of mixed cellulose ester solutions with different compositions were prepared by mixing nitrocellulose, cellulose acetate, and acetone so that the total content of nitrocellulose and cellulose acetate was 2 wt% and the weight ratios of nitrocellulose to cellulose acetate were 3:1, 1:1, and 1:3. As a comparative sample, a PMMA solution, which has been conventionally used as a transfer support film for graphene transfer, was prepared. PMMA (Sigma-Aldrich, product No. 182265) was dissolved in ethyl lactate as a solvent to prepare a PMMA solution with a PMMA content of 4 wt%.

Next, the porous membrane-forming solution was dropped onto the graphene film grown on the copper foil, and spin coating was carried out at 2000 rpm to form a nitrocellulose membrane, a cellulose acetate membrane, or a mixed cellulose ester membrane. Similarly, under the same conditions, a PMMA film as a transfer support film was formed on the graphene film grown on the copper foil. After formation of the porous membrane or the transfer support film, the unnecessary graphene film formed on the back side of the copper foil was removed by oxygen plasma treatment. Thus, a laminated body consisting of a porous membrane/graphene film/copper foil was obtained. As a comparative example, a laminated body composed of a PMMA film (transfer support film)/graphene film/copper foil was obtained.

An etching bath was prepared, and was filled with an aqueous solution of ferric nitrate (III) (0.5 mol/L) at 25 °C as a copper etchant. The size of the laminated body composed of a porous membrane/graphene film/copper foil to be subjected to copper etching was 15 mm × 10 mm. The laminated body was floated on the copper etchant with the copper foil side in contact with the solution, and the copper foil of the laminated body was removed by etching for 5 hours. Thus, a porous membrane/graphene laminated body composed of a porous membrane and a graphene film was obtained. A laminated body composed of a PMMA film (transfer support film)/graphene film was also obtained as a comparative sample.

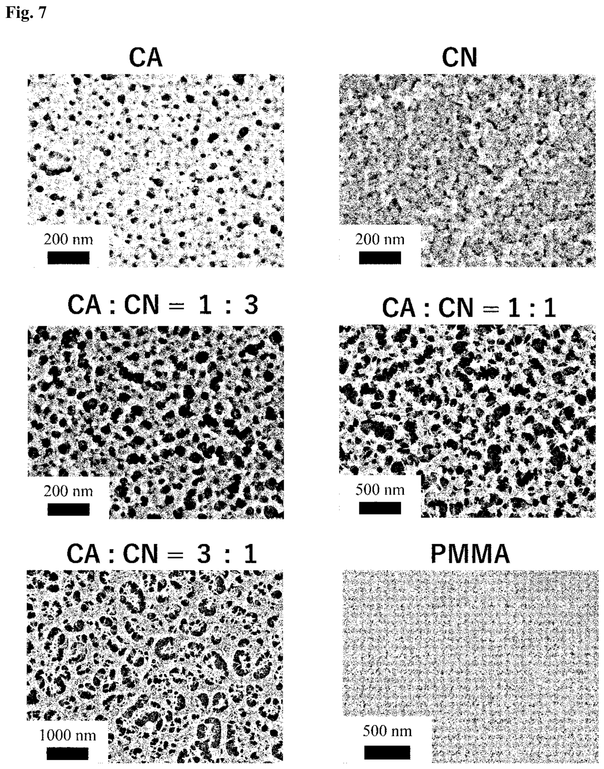

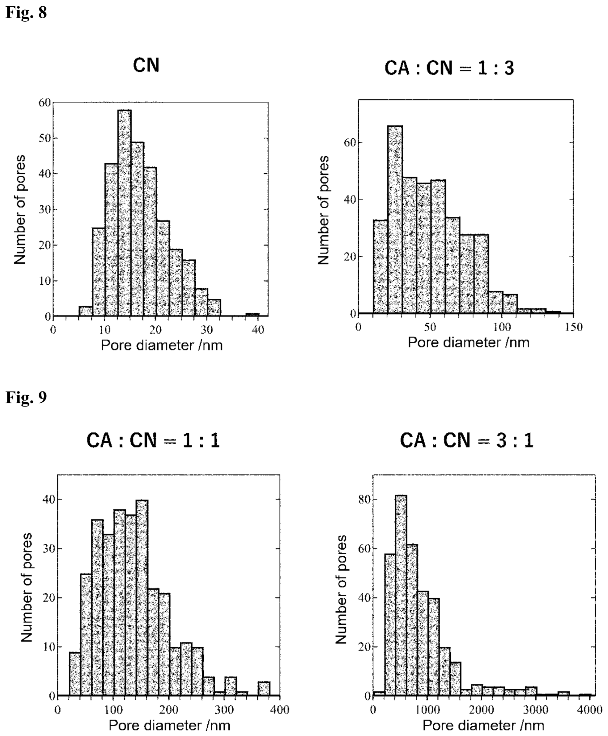

The surface of the porous membrane of the fabricated porous membrane/graphene laminated body was observed using FE-SEM (FE-SEM, manufactured by ZEISS), and the pore diameter and the fraction of the surface occupied by pores were measured from the SEM images obtained. The samples used for SEM observation were prepared by coating the surface of the porous membrane of the porous membrane/graphene laminated body formed on the copper foil after spin coating with a 3 nm platinum layer. Similarly, the surface of the PMMA film used in the comparative example was also observed. The SEM images are shown in Fig. 7. The PMMA film had a smooth surface and formed a dense film structure without pores. The CN membrane and the CA membrane had pores, but they consisted of small pores, and no pores larger than 100 nm were observed. The CA membrane had a surface with a low pore density. All the fabricated mixed cellulose ester membranes had porous structures, and it was observed that the higher the weight ratio of nitrocellulose, the larger the pore diameter tended to be. The diameters of all pores recognizable in the SEM images were estimated based on the scale bar. From these pore diameter data, the pore area ratio (the area fraction of pores relative to the surface of the porous membrane) was calculated for the CN membrane. For the mixed cellulose ester membrane, the SEM images were converted to black-and-white (binary) images, and the fraction of the area occupied by the black regions was calculated as the pore area ratio. The pore size distributions (histograms) of each sample are shown in Figs. 8 and 9. Accordingly, histograms are shown for all samples except the PMMA film (no pore) and the CA membrane (low pore density). The CN membrane had only pores with diameters of 40 nm or less, with an average pore diameter of 17 nm. The pore area ratio of the CN membrane was 2.1%. The mixed cellulose ester membrane with CN:CA = 1:3 had only pores of 150 nm or less, with an average pore diameter of 49 nm. The mixed cellulose ester membranes with CN:CA = 1:1 and CN:CA = 3:1 had pores larger than 150 nm, with average pore diameters of 140 nm and 880 nm, respectively. The CN:CA = 3:1 membrane had many large pores of around 550 nm in diameter, as well as a pore size distribution including even larger pores of 1.5 µm or more. The pore area ratio of the mixed cellulose ester membranes with CN:CA= 1:3, CN:CA = 1:1, and CN:CA= 3:1 was 38%, 40%, and 32%, respectively.

### (Example 2: Production of a laminated body composed of a porous membrane, a graphene film, and a transparent insulating substrate)

In this example, the porous membrane/graphene laminated body produced in Example 1 was transferred onto a transparent insulating substrate to produce a laminated body comprising a porous membrane, a graphene film, and a transparent insulating substrate. As the transparent insulating substrate, a quartz glass substrate (thickness: 1 mm, 20 mm × 20 mm) was prepared. On part of the quartz glass substrate, 10 nm of chromium was deposited using a vacuum deposition apparatus, and 50 nm of gold was further deposited on top of the chromium. This metal-deposited film functioned as a contact electrode when the porous membrane/graphene laminated body was laminated onto the quartz glass substrate.

A water bath filled with deionized water was prepared. After copper etching, the porous membrane/graphene laminated body floating in the etchant was transferred onto the surface of the water bath and washed. The laminated body was further transferred into another water bath to repeat the washing process. This washing step was performed three times. The porous membrane/graphene laminated body floating in the deionized water bath was scooped up using a quartz glass substrate that had been pre-deposited with chromium and gold to serve as a contact electrode. In this process, the porous membrane/graphene laminated body was laminated so that a portion of it overlapped with part of the contact electrode. In this manner, a laminated body comprising a quartz glass substrate as the transparent insulating substrate, a porous membrane, and a graphene film was produced.

The thickness of the porous membrane on the produced laminated body was measured using a stylus profilometer (manufactured by BRUKER). The thickness of the laminated body on a quartz glass substrate produced using a 2 wt% cellulose acetate and nitrocellulose mixed solution (CN:CA = 3:1) was about 400 nm. In contrast, the thickness of the laminated body produced using an ethyl lactate solution containing 4 wt% PMMA was about 150 nm.

### (Example 3: Verification of the transfer support capability of porous membranes)

In this example, the transfer support ability of the porous membrane during the etching step of the metal catalyst substrate or the subsequent transfer step to a transparent insulating substrate was verified.

Except that an acetone solution of nitrocellulose at a concentration of 1 wt% was used, a porous membrane was formed on a graphene film under the same conditions as in Example 1. The thickness of the porous membrane formed on the graphene film was less than 100 nm. When copper-foil etching was performed after porous membrane formation, tearing of the membrane occurred due to insufficient supporting strength.

Similarly, porous membranes were formed on graphene films under the same conditions as in Example 1, except that acetone solutions containing 6 wt% nitrocellulose or 4 wt% cellulose acetate were used. The porous membranes formed on the graphene films had thicknesses of 1 µm or more. Although the porous membranes were able to support the graphene film during the copper foil etching step, partial delamination from the quartz substrate was observed after transfer. This indicates that, as the membrane thickness increased, the internal stress of the support membrane also increased, exceeding the adhesion strength between the graphene and the quartz glass substrate. It is also considered that under these support membrane formation conditions, a sufficiently porous structure to relieve the stress was not generated. On the other hand, when a PET substrate was used instead of the quartz glass substrate, no delamination occurred even when the porous membrane thickness was 1 µm or more. When a support membrane with a thickness of around 1 µm was used, its greater flexibility led to stronger adhesion to the PET substrate than to the smooth surface of the quartz glass substrate.

### (Example 4: Evaluation of laminated body comprising a graphene film and a porous membrane, and electrochemiluminescence measurement)

It was verified whether the porous membrane/graphene laminated body transferred onto a quartz glass substrate, produced in Example 2, could be applied to electrochemiluminescence measurement.

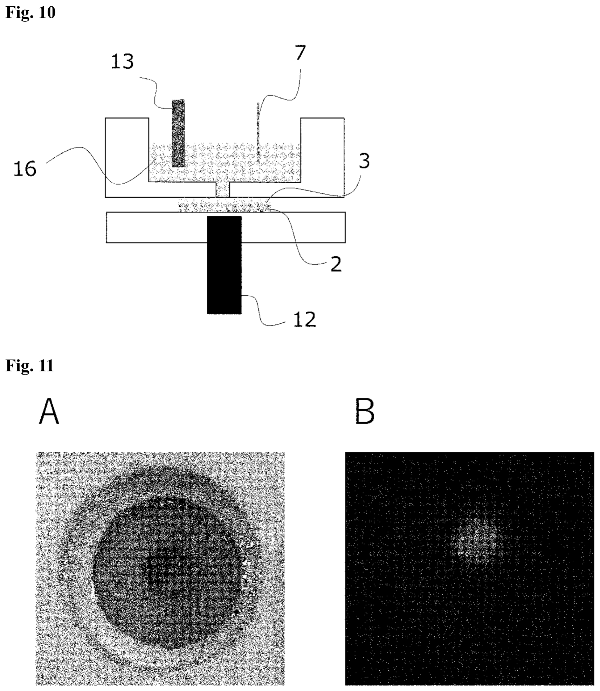

Electrochemiluminescence measurement was performed using the laminated body obtained in Example 2, comprising the porous membrane and the graphene film formed on the quartz glass substrate with a deposited film. The measurement cell shown in Fig. 10 was used for electrochemiluminescence measurement. As the working electrode, the laminated body produced in Example 2, comprising the porous membrane and the monolayer graphene film laminated on a quartz glass substrate, was used, and the Au/Cr deposited film was used as a contact electrode. As the reference electrode, an Ag/AgCl electrode (BAS Co., Ltd.) was used, and as the counter electrode, a platinum wire (0.5 mm in diameter, Nilaco) was used. In this example, an electrochemiluminescence system employing tris(2,2'-bipyridyl)ruthenium(II) as the luminescent substance and tripropylamine as the co-reactant was employed. As the electrolyte solution, a 0.15 mol/L phosphate buffer solution (pH 7.4) containing 10 mmol/L tripropylamine and 1 mmol/L tris(2,2'-bipyridyl)ruthenium(II) was used. An O-ring with a diameter of 4 mm was placed on the porous membrane/graphene laminated body, and a measurement cell was assembled by sandwiching the laminated body with PTFE resin blocks. A photomultiplier tube (H12690-300, Hamamatsu Photonics) was placed on the quartz glass substrate side of the laminated body as the photodetector for measuring electrochemiluminescence intensity.

Using the cyclic voltammetry (CV) method, the potential of the laminated body composed of the porous membrane/graphene film/transparent insulating substrate, serving as the working electrode, was swept from 0 V to 1.4 V relative to the reference electrode, while measuring the current flowing between the working and counter electrodes and the amount of electrochemiluminescence generated at the surface of the working electrode.

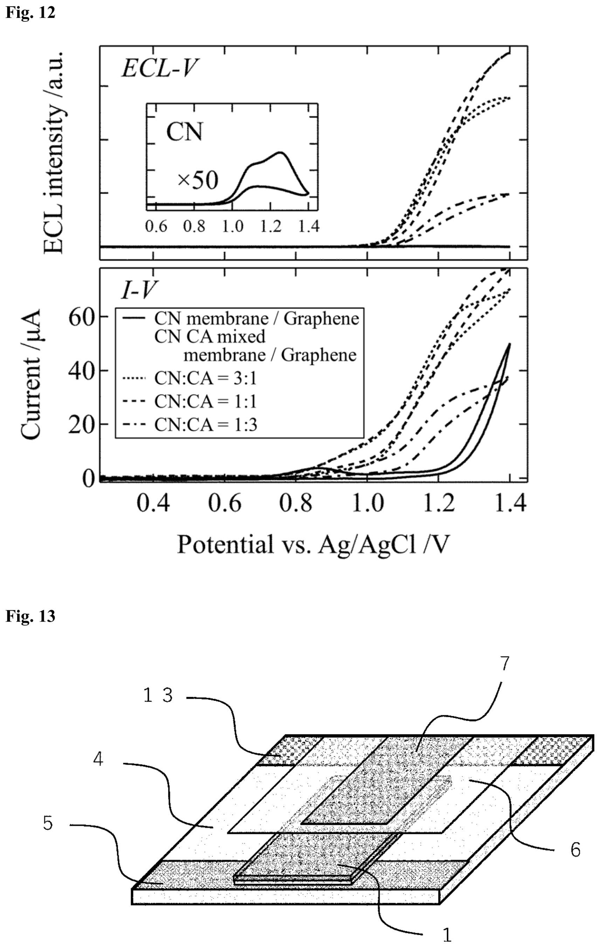

Fig. 11 shows a photograph of the electrode surface when measurement was conducted using the porous membrane/graphene laminated body comprising a mixed cellulose ester membrane with a CN:CA weight ratio of 3:1 as the working electrode, demonstrating that luminescence was observed from the entire region in contact with the electrolyte solution. These results indicate that the electrolyte solution permeated throughout the porous membrane enclosed by the O-ring, and that tripropylamine and tris(2,2'-bipyridyl)ruthenium(II) reacted on the graphene film to generate electrochemiluminescence. Fig. 12 shows the results of electrochemiluminescence measurements conducted using a nitrocellulose membrane/graphene laminated body or a mixed cellulose ester porous membrane/graphene laminated body as the working electrode. An increase in current attributed to the oxidation reaction of tripropylamine was observed from 0.8 V, and an increase in current attributed to the oxidation reaction of tris(2,2'-bipyridyl)ruthenium(II) was observed around 1.0 V. At the same time, electrochemiluminescence was confirmed to occur around 1.0 V. In the CA membrane/graphene laminated body, almost no current flowed and no electrochemiluminescence was observed. In the CN membrane/graphene laminated body, both the current and electrochemiluminescence intensity were smaller than in the mixed cellulose ester membranes. The current increase from 1.2 V in the current-potential (CV) curve of the CN membrane/graphene laminated body is considered to be due to electrolysis of water, the solvent. SEM images showed that the pore sizes of the CN membrane surface were small and the pore area ratio was low; therefore, only a small amount of luminescent substance and the co-reactant permeated through the CN membrane and reached the graphene film, resulting in lower luminescence intensity. Comparing the three mixed cellulose ester porous membrane/graphene laminated bodies, the sample with a CN:CA ratio of 1:3 as the working electrode showed lower current and electrochemiluminescence intensity than the other two samples. From the SEM images, this is considered to be due to the smaller pore size in the CN:CA = 1:3 sample compared with the other mixed cellulose ester membranes. The average pore size of the CN:CA = 1:3 sample was about 50 nm, and the porous structure consisted entirely of pores 150 nm or smaller, whereas the other two mixed cellulose ester membranes had many pores larger than 150 nm. These findings indicate that having a relatively larger pore size is preferable for enhancing electrochemiluminescence intensity.

### (Example 5: Fabrication of a three-electrode measurement chip and evaluation by electrochemiluminescence measurement)

A three-electrode measurement chip was fabricated by laminating a nitrocellulose membrane on the porous membrane/graphene laminated body laminated on a transparent insulating substrate, and further laminating a platinum counter electrode on the nitrocellulose membrane, and its applicability to electrochemiluminescence measurement was verified. An overview of the fabricated three-electrode electrochemiluminescence measurement chip is shown in Fig. 13. On the transparent insulating substrate 4, in addition to the Au/Cr deposited film serving as the contact electrode for the porous membrane/graphene laminated body 1, a silver/gold/chromium deposited film (silver electrode) was formed. The fabricated electrochemiluminescence measurement chip comprised a three-electrode system consisting of the porous membrane/graphene laminated body 1 as the working electrode, the silver electrode as the reference electrode 13, and the platinum electrode (electrical conductor 7) as the counter electrode.

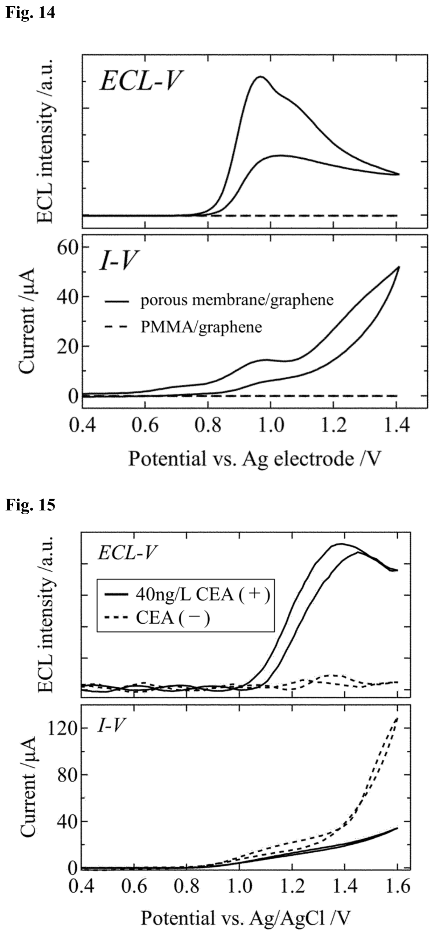

As in Example 1, a porous membrane/graphene laminated body was fabricated and transferred onto a quartz glass substrate deposited with chromium and gold as in Example 2. The porous membrane was formed on the graphene film by spin coating, using an acetone solution containing nitrocellulose and cellulose acetate (CN:CA = 3:1) at a total concentration of 2 wt%. In this example, in addition to the Au/Cr deposited film, a silver/gold/chromium deposited film was formed on the quartz glass substrate. During transfer of the porous membrane/graphene laminated body onto the quartz glass substrate, the laminated body was scooped up onto the quartz glass substrate so that it overlapped with the Au/Cr deposited film but not with the silver/gold/chromium deposited film. Subsequently, a nitrocellulose membrane (Cytiva, AE98, without backing sheet, thickness: 105-140 µm) was stacked on the quartz glass substrate so as to overlap both the porous membrane/graphene laminated body and the silver/gold/chromium deposited film. A platinum counter electrode was further laminated on the nitrocellulose membrane to produce a three-electrode measurement chip. The silver/gold/chromium deposited film on the quartz glass substrate was used as the reference electrode for electrochemiluminescence measurement. The nitrocellulose membrane was impregnated with a phosphate buffer solution (pH 7.4) containing 10 mmol/L tripropylamine and 1 mmol/L tris(2,2'-bipyridyl)ruthenium(II), and CV measurement was carried out. Conditions other than the cell structure used for the measurement were the same as in Example 4. The area of the working electrode was set at 1 cm². For comparison, a three-electrode measurement chip was also fabricated using a PMMA film/graphene laminated body, conventionally used as a transfer support film, as the working electrode, and electrochemiluminescence measurement was conducted. Fig. 14 shows the results of the electrochemiluminescence measurements. In the measurement chip using the mixed cellulose ester porous membrane/graphene laminated body as the working electrode, a current attributed to the oxidation of tripropylamine was observed from 0.6 V, and an increase in current attributed to the oxidation of tris(2,2'-bipyridyl)ruthenium(II) was observed from 0.8 V. Correspondingly, an increase in luminescence intensity was observed from 0.8 V. In contrast, when a measurement chip using a PMMA/graphene laminated body as the working electrode was employed, neither current nor electrochemiluminescence was observed. As shown in the SEM image in Fig. 7, this is because the fabricated PMMA film had a dense film structure, preventing penetration of the electrolyte solution into the PMMA film and thus no electrode reaction occurred. These results confirmed that a three-electrode measurement chip using a porous membrane/graphene laminated body as the working electrode can be applied to electrochemiluminescence measurement.

### (Example 6: Electrochemiluminescence immunoassay employing porous membrane/graphene laminated body)

In this example, to verify whether the porous membrane/graphene laminated body according to the present invention could be used as an electrode for electrochemiluminescence immunoassay, the detection of carcinoembryonic antigen (CEA) was attempted using an electrochemiluminescence immunoassay method with the porous membrane/graphene laminated body.

Specifically, an anti-CEA antibody was immobilized on the porous membrane of the porous membrane/graphene laminated body, and a solution containing CEA as the antigen and an anti-CEA antibody labeled with tris(2,2'-bipyridyl)ruthenium(II) as the labeling substance was dropped onto the porous membrane to form a sandwich-type immune complex on the porous membrane of the laminated body through an antigen-antibody reaction. A phosphate buffer solution (0.15 mol/L) containing 10 mmol/L tripropylamine, a co-reactant of tris(2,2'-bipyridyl)ruthenium(II), was used as the electrolyte solution. It was verified that immunoassay could be performed by observing electrochemiluminescence generated by the oxidation of tripropylamine on the electrode and subsequent reaction with the tris(2,2'-bipyridyl)ruthenium(II) bound as a label in the immune complex.

### 6-1. Reagents

- Elecsys Reagent CEA II (containing biotinylated anti-CEA antibody and tris(2,2'-bipyridyl)ruthenium(II)-labeled anti-CEA antibody) (Roche Diagnostics, Cat. No. 570444-016)
- CEA Calibrator (containing CEA) (Roche Diagnostics, Cat. No. 316457)
- Blocking Reagent N101 (NOF Corporation)

### 6-2. Fabrication of laminated body

Nitrocellulose (CN) and cellulose acetate (CA) were dissolved in acetone at a weight ratio of CN:CA= 3:1 and a total solute concentration of 2 wt% to prepare a solution for forming the porous membrane.

Using the prepared porous-membrane-forming solution, a laminated body comprising a graphene film and a porous membrane was fabricated under the same conditions as in Example 1, and transferred onto a quartz glass substrate (20 mm × 20 mm × 1 mm). Before transfer, the quartz glass substrate was cleaned using the same procedure as for cleaning the copper foil used for CVD formation. The quartz glass substrate was pre-equipped with a deposited electrode composed of chromium and gold as a contact electrode. In the following electrochemiluminescence immunoassay, the laminated body transferred onto the quartz glass substrate was used as the working electrode.

### 6-3. Electrochemiluminescence immunoassay

A solution containing 3 mg/L of biotinylated anti-CEA antibody (hereinafter, "capture antibody") was dropped (20 µL) onto the porous membrane of the porous membrane/graphene laminated body (hereinafter, "electrode") on the quartz glass substrate, and the electrode was placed in a sealed container to prevent drying and incubated at 37°C for 60 minutes. After the 60-minute incubation, the solution containing the capture antibody on the porous membrane was blotted with nonwoven fabric, and the electrode was immersed in blocking solution and incubated at 37 °C for 30 minutes. The blocking solution used was a five-fold dilution of Blocking Reagent N101 with pure water. After removal from the blocking solution, the electrode was immersed in pure water for 60 seconds, repeated twice, to wash away the blocking solution remaining on the electrode. After natural drying, 3 µL of a sample solution containing 40 ng/L CEA and 15 µL of a solution containing 4 mg/L of tris(2,2'-bipyridyl)ruthenium(II)-labeled anti-CEA antibody (hereinafter, "labeled antibody") were dropped onto the porous membrane of the electrode. The electrode was placed in a sealed container and incubated 37 °C for 90 minutes to form a sandwich-type immune complex on the porous membrane through an antigen-antibody reaction. As a negative control, an electrode prepared by immobilizing the capture antibody and performing blocking treatment in the same manner, but dropping only the labeled antibody without CEA, was prepared. After removal from the sealed container, the electrodes were immersed in pure water for 60 seconds, repeated twice, to remove residual unbound labeled antibody and CEA.

Electrochemiluminescence measurement was performed by sweeping the electrode potential from 0 V to 1.6 V using cyclic voltammetry with the same apparatus as in Example 2. As the electrolyte solution, a phosphate buffer solution (0.15 mol/L, pH 7.4) containing 10 mmol/L tripropylamine was used.

### 6-4. Results

The results of the electrochemiluminescence immunoassay are shown in Fig. 15. As shown in Fig. 15, an increase in current due to the oxidation of tripropylamine was observed when the potential was swept in the positive direction. In the sample to which CEA was added, an increase in luminescence intensity corresponding to the increase in current was observed.

### 6-5. Comparative test and results

To confirm that the above immunoassay results were due to luminescence from the immune complex of capture antibody-CEA-labeled antibody immobilized on the porous membrane, rather than luminescence from unbound labeled antibody, electrochemiluminescence measurement was performed under the same conditions using an electrode prepared by dropping only the labeled antibody onto the porous membrane with immobilized capture antibody. The results are shown by the dashed line in Fig. 15. As shown in Fig. 15, no luminescence was observed in the electrode without immobilized immune complexes. Therefore, it was confirmed that the luminescence was derived from the labeled antibody bound together with CEA to the capture antibody on the porous membrane, and not from unbound labeled antibody.

Thus, the laminated body according to the present invention can be used for electrochemiluminescence immunoassay.

### (Example 7: Evaluation of durability of the porous membrane/graphene laminated body)

When monolayer graphene is used as an electrode for electrochemical analysis, a substrate is required to support the single atomic layer of graphene. Since the adhesion between the substrate and graphene relies only on van der Waals forces acting between them, partial tearing of the graphene film may occur when the fluid in contact with the graphene flows strongly or when the solution is replaced with another of different composition, allowing the solution to penetrate between the substrate and graphene. Once tearing occurs in monolayer graphene, electrical conductivity decreases, leading to a decrease in current. Since the porous membrane/graphene laminated body according to the present invention has a porous membrane formed on the solution side, it is considered to be less prone to delamination or tearing compared to conventional graphene electrodes without a porous membrane. Therefore, in this example, the durability of the porous membrane/graphene laminated body was evaluated by repeated electrochemiluminescence (ECL) measurements. For comparison, the same test was also performed using a conventional graphene electrode without a porous membrane.

### 7-1. Fabrication of laminated body and ECL measurement

As the porous membrane/graphene laminated body, a graphene electrode transferred with a mixed cellulose ester membrane (CN:CA = 3:1) prepared by spin coating was used. The fabrication procedure was the same as in Example 2. A graphene electrode prepared by the conventional PMMA support transfer method was also used as in Example 2.

Using these electrodes, ECL measurements were conducted by cyclic voltammetry (CV) with a phosphate buffer solution (0.15 mol/L, pH 7.4) containing 10 mmol/L tripropylamine and 0.1 mmol/L tris(2,2'-bipyridyl)ruthenium(II) as the electrolyte solution. Measurements were repeated 10 times for each electrode, and after each measurement the electrode was washed with PBS and the solution was replaced. In addition, the sum of the solution resistance and the graphene resistance (Rs) was measured by electrochemical impedance spectroscopy (EIS) before measurement, and after the 5th and 10th measurements.

### 7-2. Results

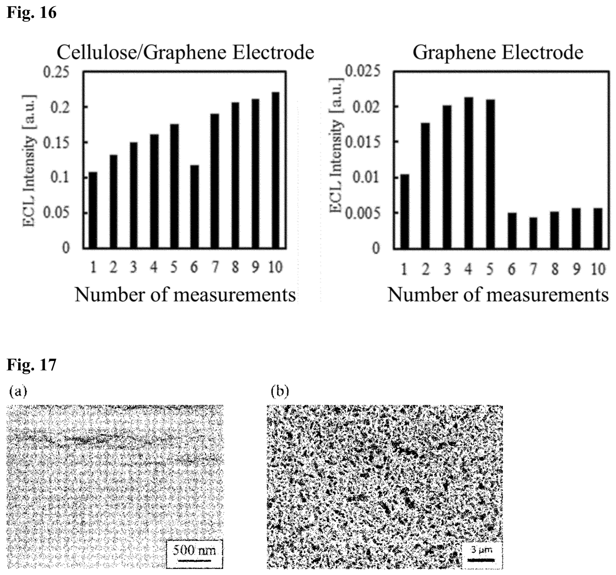

Fig. 16 shows the ECL intensity at 1.2 V for each measurement when the ECL measurement was repeated 10 times for each electrode. The ECL intensity increased up to the 5th measurement, which is considered to be due to gradual oxidation of the graphene surface, resulting in changes in the surface condition. After the 6th measurement, the ECL intensity of the graphene electrode rapidly decreased, which is considered to result from partial tearing of the graphene electrode. On the other hand, in the electrode fabricated using the cellulose membrane/graphene laminated body (cellulose/graphene electrode), although a temporary decrease in ECL intensity was observed at the 6th measurement, high ECL intensity was maintained from the 7th measurement onward. Table 1 shows the Rs values measured by EIS. Rs increased with repeated ECL measurements, which is attributed to an increase in the resistance of the graphene film. This increase is considered to result from partial oxidation of the graphene film due to water oxidation as a side reaction, as well as mechanical damage to graphene during solution replacement. Since the cellulose/graphene electrode showed a smaller degree of Rs increase, it suggests that mechanical damage such as tearing did not occur in the cellulose/graphene electrode. From these results, it is considered that the mixed cellulose ester porous membrane protected the graphene and improved durability.

**Table 1**

| Electrode | Before 1st measurement | After 5th measurement | After 10th measurement |
|---|---|---|---|
| Graphene | 768 Ω | 1420 Ω | 3220 Ω |
| Cellulose/Graphene | 459 Ω | 682 Ω | 901 Ω |

### (Example 8. Fabrication of a porous membrane/graphene laminated body by solution-casting method)

When using a porous membrane/transparent conductive film laminated body electrode in ECL immunoassay, it is preferable that the amount of antibody immobilized in the porous membrane is high. In this example, in order to increase the amount of antibody immobilized in the porous membrane, a mixed cellulose ester membrane was fabricated by the solution-casting method using an applicator, and methods for rendering the mixed cellulose ester membrane porous were investigated.

### 8-1. Examination of solvents

In this Example, a mixed cellulose ester membrane was formed using a solution casting method with an applicator, and methods for rendering the mixed cellulose ester membrane porous were examined. The mixed cellulose ester membrane used in this Example was prepared at a weight ratio of CA:CN = 1:2. First, solvents were examined. A porous membrane-forming solution was prepared by dissolving 4 wt% mixed cellulose ester in two types of solvents: acetone, and a mixed solvent of acetone and formamide (weight ratio 1:1). The solution was cast manually onto a copper substrate using an applicator with a gap height of 75 µm. Fig. 17 shows SEM images of the surface of the mixed cellulose ester membrane after drying following solution casting (platinum-coated to suppress charging). When acetone was used as the solvent, no pore structure was observed (Fig. 17(a)), whereas when the acetone/formamide mixed solvent was used, a pore structure was observed (Fig. 17(b)). Acetone is a good solvent for both CA and CN, whereas formamide is a good solvent only for CN and a poor solvent for CA. In the acetone/formamide mixed solvent, as the good solvent acetone evaporates during casting, the solvent quality of the solution gradually becomes poorer, and phase separation occurs. It is considered that porosity of the membrane was introduced in the acetone/formamide mixed solvent by this phase separation.

### 8-2. Examination of phase inversion method and SEM observation

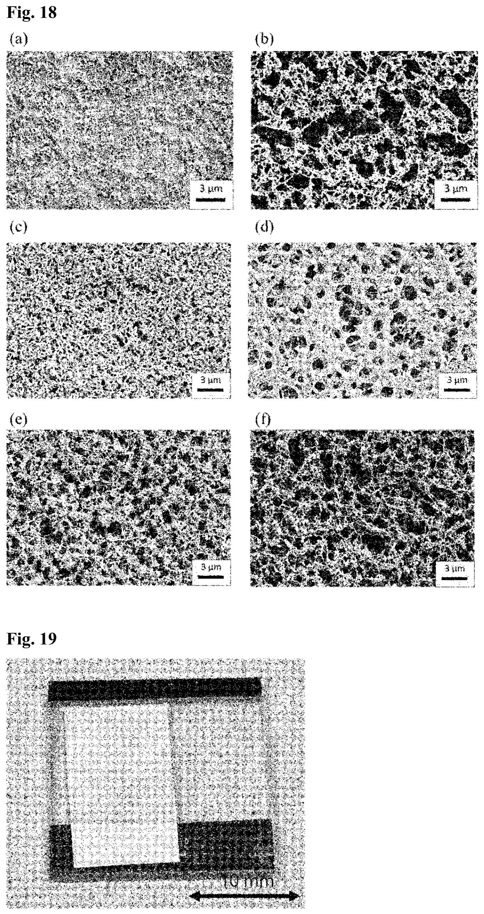

Next, a method of immersing the cast membranes in ultrapure water to induce porosity by phase inversion was investigated. At the same time, the effect of solute concentration of the mixed cellulose ester solution on the porous structure was also evaluated. Mixed cellulose ester-forming solutions were prepared by dissolving CN and CA in an acetone/formamide mixed solvent (weight ratio 1:1) at concentrations of 4, 8, and 12 wt%. The solutions were cast manually onto copper substrates using an applicator with a gap height of 75 µm. For each concentration, two types of samples were prepared: one dried immediately after casting, and one immersed in ultrapure water for 24 hours immediately after casting and then dried. The surfaces of the fabricated mixed cellulose ester membranes were platinum-coated, and their surface structures were observed by SEM. Fig. 18 shows SEM images of the surfaces of six mixed cellulose ester membranes prepared: (a) 4 wt% without immersion, (b) 4 wt% with immersion, (c) 8 wt% without immersion, (d) 8 wt% with immersion, (e) 12 wt% without immersion, and (f) 12 wt% with immersion. In all solute concentrations, it was observed that immersion in water after casting resulted in an increase in pore diameter. This is because immersion in water caused gelation of the casting solution, resulting in replacement of the solvent of the casting solution with water, and phase separation occurred.

### 8-3. Film thickness measurement

Table 2 shows the thickness of the porous membranes measured by a Dektak profiler.

**Table 2**

| Solute concentration | Water immersion | Film thickness (µm) |
|---|---|---|
| 4 wt% | No | 1.0 |
| 4 wt% | Yes | 2.7 |
| 8 wt% | No | 1.3 |
| 8 wt% | Yes | 5.5 |
| 12 wt% | No | 3.5 |
| 12 wt% | Yes | 16 |

From the film thickness measurement results, it was observed that both increasing solute concentration and immersion in water caused an increase in film thickness. Since immersion in water significantly increased the thickness of the porous membrane, it indicates that the immersed samples contained many voids. With increasing solute concentration, the amount of solute contained in the casting solution increased, which also resulted in increased film thickness. In the 12 wt% sample immersed in water, the remarkable increase in film thickness is considered to be related to the viscosity of the porous membrane-forming solution. As the viscosity increased, the fluidity of the solute decreased and aggregation of the solute was suppressed, thereby reducing shrinkage of the porous membrane thickness relative to the applicator gap. Table 3 shows the viscosity of the porous membrane-forming solutions measured with a viscometer (Anton Paar, model ViscoQC 100, spindle SC4-21).

**Table 3**

| Solute concentration | Speed /rpm | Viscosity /mPa·s | Torque /% |
|---|---|---|---|
| 4 wt% | 50 | 37 | 23.1 |
| 8 wt% | 50 | 138 | 86.4 |
| 12 wt% | 12 | 609 | 91.4 |

### 8-4. Transfer to a Substrate

Next, whether graphene grown on a copper foil could be transferred onto a quartz glass substrate using these mixed cellulose ester membranes was examined. Using mixed cellulose ester membranes prepared with an acetone solvent solution, as prepared in Example 8-1, transfer of graphene to a quartz glass substrate was attempted according to the same procedure as in Example 2. As a result, these laminated bodies peeled off from the quartz glass substrate during the drying process after transfer and could not be transferred onto the quartz glass substrate. On the other hand, all samples prepared from porous-membrane-forming solutions using acetone/formamide mixed solvents were successfully transferred. In the case of films prepared using an applicator, the increased thickness compared with spin coating was considered to also increase tensile stress. In the case of mixed cellulose ester membranes prepared from an acetone solvent solution, no porous structure was formed, whereas in the acetone/formamide mixed solvent, a porous structure was formed throughout the membrane, which is considered to have relieved the tensile stress and prevented delamination. A photograph of a porous mixed cellulose ester film/graphene film/quartz glass substrate laminated body transferred onto a quartz glass substrate using a 12 wt% support membrane immersed in water is shown in Fig. 19. Thus, it was found that by making the support film porous, even when the thickness was 10 µm or more, the support film/graphene laminated body could be transferred onto a substrate without delamination.

### 8-4. Raman Spectroscopy

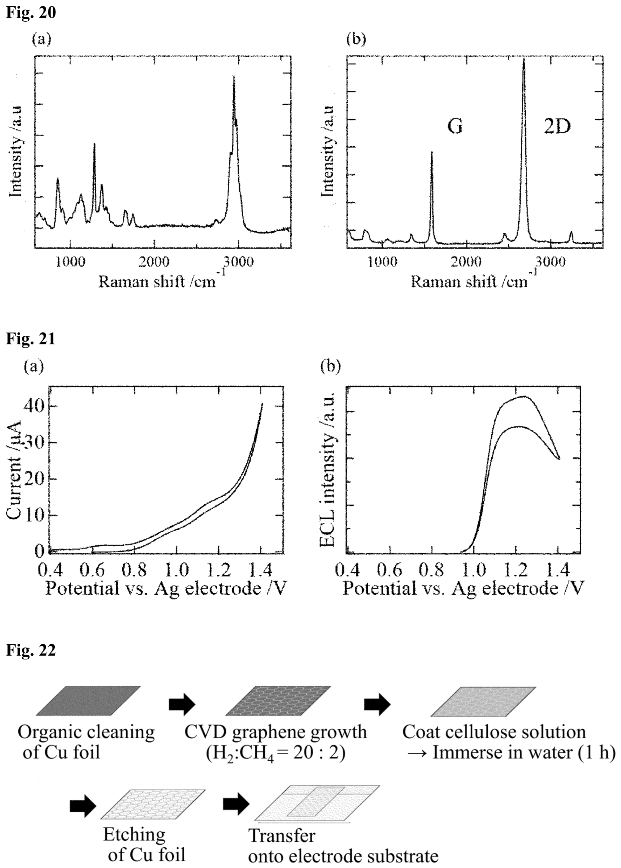

Figure 20(a) shows the Raman spectrum of the sample shown in Figure 19. Peaks attributable to CH₃ and C-H of cellulose, appearing around 1280 cm⁻¹ and 2950 cm⁻¹, were observed; however, the 2D peak and G peak attributable to graphene were not observed. This is considered to be because the support film was porous and thick, and thus the laser light was scattered by the support film and did not reach the graphene. Subsequently, Raman spectroscopy was carried out after immersing the mixed cellulose ester/graphene laminated body in heated acetone for 20 minutes to remove the mixed cellulose ester membrane serving as the support film. Fig. 20(b) shows the Raman spectrum of the graphene after removal of the support film. The 2D peak and G peak attributable to graphene were observed, and the D peak around 1350 cm⁻¹, which indicates defects, was hardly observed, indicating that high-quality graphene could be produced even when a mixed cellulose ester membrane prepared by the solution casting method was used as a support material. Furthermore, since the peak attributable to the C-H of cellulose appearing around 2950 cm⁻¹ was not observed, it was confirmed that the mixed cellulose ester was removed by immersion in heated acetone (50 °C).

### 8-5. Electrical Resistance Measurement

The sheet resistance of the porous mixed cellulose ester/graphene film/quartz glass substrate laminated body transferred by the same procedure as the sample shown in Fig. 19 was measured by the van der Pauw method, and the result was 560 Ω/sq.

For comparison, the sheet resistance of graphene prepared by conventional PMMA-assisted transfer and after removal of the PMMA film was about 550 Ω/sq. This shows that graphene transferred using mixed cellulose ester films prepared by the solution-casting method is of similarly high quality as that prepared by the PMMA-assisted transfer method.

### 8-6. Electrochemiluminescence Measurement

Fig. 21 shows the results of electrochemiluminescence (ECL) measurement using a mixed cellulose ester/graphene film/quartz glass substrate laminated body prepared by the same procedure as the sample shown in Fig. 19 as an electrode. The measurement solution was a phosphate buffer (pH 7.4) containing 10 mmol/L tripropylamine and 0.1 mmol/L ruthenium bipyridyl complex. In the current-potential curve shown in Fig. 21(a), a current due to the oxidation reaction of tripropylamine started to flow around 0.8 V, and in the ECL curve shown in Fig. 21(b), an increase in ECL was observed around 1.0 V.

These results indicate that the pore structure of the mixed cellulose ester extended continuously to the graphene, allowing reactants in the electrolyte solution to sufficiently reach the graphene and undergo electrochemical reactions. Based on the results of SEM observation, Raman spectroscopy, electrical resistance measurements, and ECL measurements, it was demonstrated that mixed cellulose ester membranes prepared by the solution-casting method exhibit continuous porous structures extending from the surface to the interior and down to the graphene film, even when the film thickness is 10 µm or more.

### (Example 9. Protein adsorption capacity of porous membranes formed by the solution-casting method)

In this example, to enhance luminescence intensity in ECL immunoassay, it was examined whether increasing the thickness of mixed cellulose ester membranes could increase the antibody loading capacity of porous mixed cellulose ester/graphene laminated films.

In this example, an applicator was used to increase the thickness of the porous membranes. Furthermore, since it is difficult for cellulose membranes to form a porous structure when the cellulose solution is applied thickly, a process of immersing the coated cellulose solution in water to replace the solvent with water (a wet phase inversion method) was incorporated. The fabrication process of porous cellulose membrane/graphene film laminated bodies by the phase inversion method is shown in Fig. 22. The cellulose solution used for forming porous cellulose membranes was prepared by dissolving cellulose acetate and nitrocellulose at a weight ratio of 1:2 in an acetone/formamide mixed solvent (1:1 by weight) to a concentration of 12 wt% (609 mPa·s). In addition, by varying the applicator gap height to 25 µm, 50 µm, 75 µm, and 100 µm, four types of porous mixed cellulose ester membranes with different thicknesses were formed. The applicator gap width was 60 mm. A fully automatic linear motion coater (ALC-mini2, COTEC Co.) capable of controlling coating speed was used to apply the solution with the applicator.

### 9-1. Preparation of laminated bodies

Specifically, laminated bodies were prepared as follows. Graphene was grown on a copper foil in the same manner as in Example 8. In this example, the coating speed during film formation with the applicator was set to 2 mm/s, and four types of porous mixed cellulose ester membranes with different thicknesses were formed on the graphene. After applying the porous-membrane-forming solution onto the graphene film, the obtained laminated body was transferred to a pure water bath and immersed for one hour. Subsequently, the laminated body was removed from the pure water and naturally dried, thereby obtaining a laminated body composed of a porous cellulose membrane/graphene film/copper foil.

### 9-2. Surface structure analysis of laminated bodies

To analyze the surface structure of the porous cellulose membrane/graphene laminated bodies formed on copper foil, the membrane surfaces were observed by scanning electron microscopy in the same manner as in Example 1, and the average pore diameter and pore area ratio were calculated from the captured images. In order to investigate how the actual membrane thickness varied with changes in the applicator gap height, membrane thickness was measured using a laser microscope.

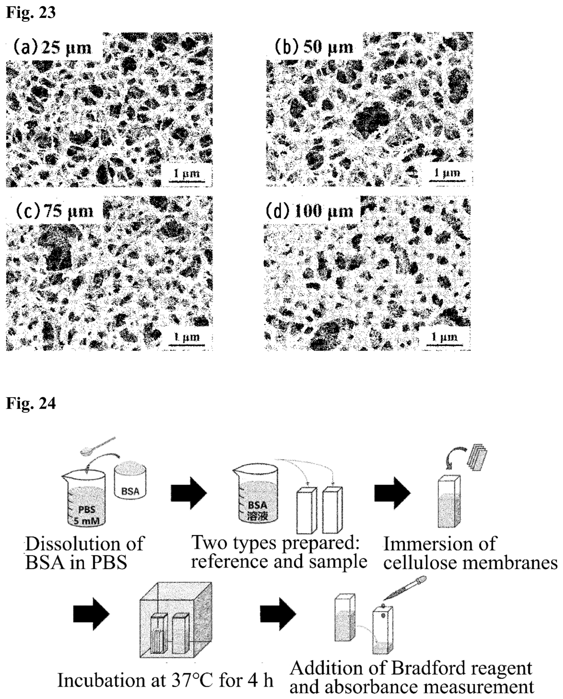

Fig. 23 shows SEM images of cellulose membranes fabricated with varying thicknesses under the condition of CA:CN = 1:2 (Fig. 23(a): 25 µm, (b): 50 µm, (c): 75 µm, (d): 100 µm). The four samples were prepared by setting the applicator gap height to 25 µm, 50 µm, 75 µm, and 100 µm, respectively. Comparison of these samples revealed that the cellulose fiber structure became thicker as membrane thickness increased. Table 4 shows the membrane thickness, mean pore diameter on the membrane surface, and pore area ratio.

**Table 4**

| Sample | Gap height /nm | Film thickness /µm | Surface average pore diameter /nm | Pore area ratio /% |
|---|---|---|---|---|
| (a) | 25 | 2.6 | 270 | 26 |
| (b) | 50 | 6.5 | 271 | 24 |
| (c) | 75 | 11.0 | 253 | 21 |
| (d) | 100 | 18.5 | 213 | 11 |

When the four samples were compared, it was observed that the membrane thickness increased with the applicator gap height. In addition, as the membrane thickness increased, the average pore size decreased, and the pore area ratio also decreased. This is considered to be because, when the cellulose solution was applied with an applicator and then immersed in water, solvent replacement was less efficient in thicker membranes, making it more difficult for voids to form upon drying.

### 9-3. Measurement of protein adsorption capacity

In order to evaluate the antibody loading capacity of the porous cellulose membranes in the fabricated laminated bodies, the protein adsorption amount of the porous cellulose membranes was quantified. The measurement of protein adsorption was performed using the Bradford reagent (APRO Science, product code: KY-1020), which changes its absorbance at 595 nm in proportion to the protein concentration when added to a protein-containing solution. First, bovine serum albumin (BSA; FUJIFILM Wako Pure Chemical Corporation, product code: 017-15146) was dissolved in 5 mmol/L PBS, and BSA solutions having five known concentrations (1000 µg/mL, 500 µg/mL, 250 µg/mL, 125 µg/mL, and 100 µg/mL) were prepared. The Bradford reagent was then added to each solution, and the absorbance at 595 nm was measured to prepare a calibration curve. Thereafter, as shown in Fig. 24, cellulose membranes were immersed in a 1000 µg/mL BSA solution, and the BSA concentrations before and after immersion were calculated from the calibration curve obtained based on the absorbance values. In this manner, the protein adsorption amount per projected area of the cellulose membrane was quantified. For comparison of protein adsorption amount, a similar experiment was also carried out using a commercially available nitrocellulose membrane for immunochromatography (Cytiva, AE98, without backing sheet, thickness: 105-140 µm).

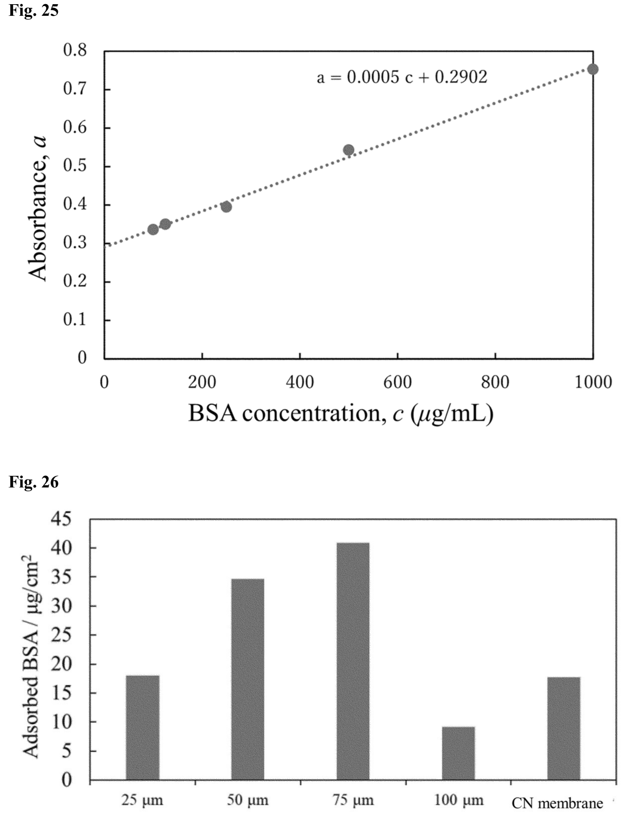

The absorbance at 595 nm was measured after adding the Bradford reagent to BSA solutions of known concentration. A calibration curve prepared based on these results is shown in Fig. 25. From Fig. 25, when the absorbance values at each concentration were plotted on the graph, a calibration curve showing high linearity was obtained. By using the equation of this approximate straight line, the concentration of BSA in solutions of unknown concentration was measured from the absorbance values obtained after the addition of the Bradford reagent.

The quantitative results of protein adsorption measured using the four cellulose membranes fabricated with different applicator gap heights and the commercial nitrocellulose membrane are shown in Fig. 26. Comparing the membranes fabricated with applicator gap heights of 25 µm, 50 µm, and 75 µm, the amount of BSA adsorption increased with increasing membrane thickness. In contrast, for the membrane fabricated with an applicator gap height of 100 µm, the amount of BSA adsorption decreased. As shown in Table 4, this was attributed to the thickening of the cellulose fiber structure with increasing membrane thickness, leading to reduced void volume inside the membrane and thus a smaller internal surface area available for BSA adsorption. From these results, it was concluded that membranes fabricated under conditions with applicator gap heights in the range of 25 µm to 75 µm are suitable for use as cellulose membranes in ECL immunoassay applications. Furthermore, comparison with the commercial nitrocellulose membrane showed that, despite having less than one-tenth of the thickness, the protein adsorption capacity reached twice that of the commercial nitrocellulose membrane.

### (Example 10. Fabrication of a laminated body comprising a porous membrane/graphene film with compressed porous regions)

When using a laminated electrode comprising a porous membrane and a transparent conductive film as an analytical electrode, for example, for a biochip, it is desirable to define the area of graphene exposed to the solution. Since the sheet resistance of monolayer graphene is on the order of several hundred Ω/sq, when a high current flows, ohmic loss occurs within the graphene electrode, and the electrode reaction becomes limited by the electrical resistance of the graphene. Therefore, the electrode area should be appropriately defined so that the ohmic loss does not become excessive. Furthermore, when using graphene as an electrode, it is necessary to laminate the edge of the graphene sheet with a contact electrode. Since it is also necessary to prevent the contact electrode from coming into contact with the electrolyte solution, one conceivable method for defining the electrode area is to laminate an insulating material on part of the graphene electrode surface so as to limit the area in contact with the electrolyte solution. However, when the porous membrane of a laminated electrode composed of a porous membrane and a transparent conductive film increases in thickness, even if an insulating material is placed on the porous membrane surface, the electrolyte solution permeates through the porous membrane, making it difficult to restrict the graphene electrode area exposed to the solution. Therefore, in this example, pressure was applied perpendicular to the surface of the laminated electrode to compress the porous membrane, except for the region intended to be in contact with the solution, thereby collapsing the porous structure. This was done to prevent horizontal movement of the electrolyte solution and to investigate whether the compressed porous membrane could serve as an insulating material for defining the electrode area. To avoid damaging the graphene during compression of the laminated electrode, the electrical resistance of the laminated body was used as an indicator to determine the appropriate pressure for compressing the porous structure. The surfaces of the compressed and uncompressed regions of the porous membrane were observed by SEM.

### 10-1. Fabrication of laminated bodies comprising porous membrane/graphene film with compressed porous regions

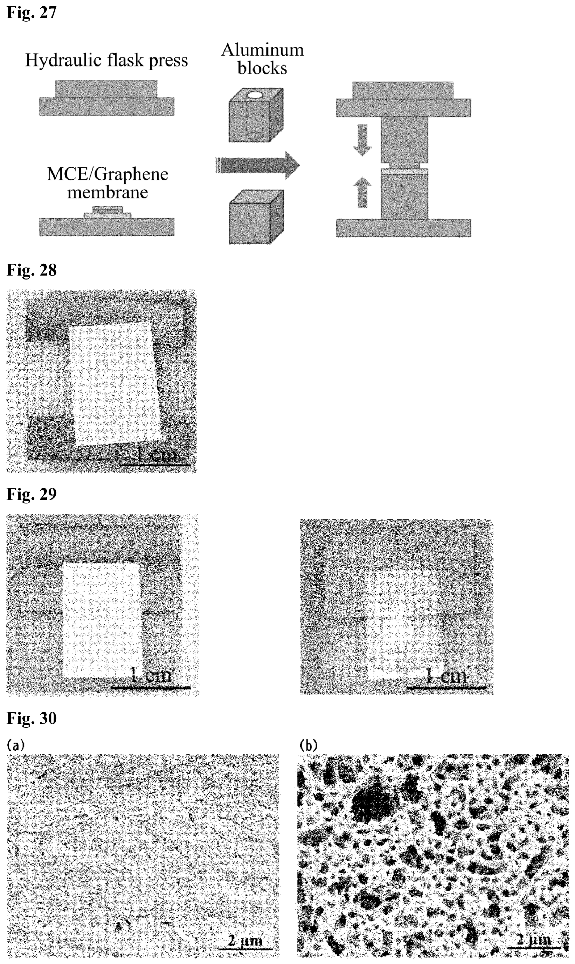

A porous mixed cellulose ester membrane was formed on graphene grown on copper under the same conditions as in Example 9 with an applicator gap height of 75 µm. Similarly, using the procedure described in Example 2, a laminated body comprising a porous mixed cellulose ester membrane and a graphene film was transferred onto a quartz glass substrate. Portions of the porous structures of these two types of laminated bodies (on copper foil or on quartz glass substrate) were compressed using a hydraulic flask press (FP7, Morita Tokyo Mfg. Co., Ltd.) and aluminum blocks, as shown in Fig. 27. The surface of the aluminum block in contact with the sample was 20 mm × 20 mm in size. When leaving non-compressed regions, 20 mm × 20 mm aluminum plates having holes with a diameter (Φ) of 4 mm or 8 mm were used on the porous membrane side.

### 10-2. Application of pressure to the entire laminated body

First, as shown in Fig. 28, a laminated body (15 mm × 10 mm, quartz glass substrate 20 mm × 20 mm, channel area 10 mm × 10 mm) comprising a porous mixed cellulose ester membrane/graphene film on a quartz glass substrate with gold/chromium deposited at both ends was subjected to pressure using a 20 mm × 20 mm aluminum block (without holes). The change in electrical resistance between the gold/chromium electrodes at both ends (corresponding to the resistance of the graphene film) was examined. A total of eight applications of force were performed on the laminated body. The applied force was varied for each measurement. Table 5 shows the relationship between the applied force on the porous membrane/graphene laminated body and the electrical resistance.

**Table 5**

| | | | | | |
|---|---|---|---|---|---|
| Number of compressions [times] | 0 | 1 | 4 | 6 | 8 |
| Resistance [Ω] | 400 | 400 | 420 | 780 | 2200 |
| Applied Force [N] | 0 | 5 × 10³ | 1 × 10⁴ | 1 × 10⁴ | 1.5 × 10⁴ |

The "Applied Force [N]" in Table 5 refers to the force applied to the porous membrane/graphene electrode using a hydraulic press (flask press). The resistance of the laminated electrode before applying force was 400 Ω. When a force of 5 kN was applied in the first trial, no change in resistance was observed. Furthermore, when the porous structure was collapsed with a force of 10 kN (approximately 1 ton) in the fourth trial, the resistance increased slightly to 420 Ω, but this change was not large enough to impair electrode performance. This indicates that the porous membrane/graphene laminated electrode has high durability against pressure applied from above and below. It was found that applying a force of about 1 ton (250 MPa) did not impair electrode performance. Thereafter, the porous membrane was compressed repeatedly until the electrode ceased to function. When the porous structure was collapsed with a force of 1 ton in the sixth trial, the resistance further increased, and when a force of 1.5 tons was applied in the eighth trial, the resistance rose to 2200 Ω, indicating a significant deterioration in electrode performance. Since cracks were observed in the quartz glass substrate at this point, it is considered that breakage of the quartz glass substrate was the cause of the large increase in resistance.

### 10-3. Application of partial pressure to the laminated body

Next, as shown in Fig. 29, a force of 1 ton was applied for 30 seconds to a laminated electrode on a quartz glass substrate using an aluminum block with a 4 mm diameter hole. In the photograph taken before applying force (left image in Fig. 29), the porous structure of the mixed cellulose ester membrane scattered light, causing it to appear white, whereas in the photograph taken after applying force (right image in Fig. 29), the region in contact with the aluminum block appeared transparent. This is believed to be because the porous structure was compressed and no longer scattered light.

### 10-4. SEM observation

To investigate whether the porous structure of the cellulose membrane was collapsed after applying force, a laminated body formed on copper foil was compressed with a force of 1.25 tons using an aluminum block with an 8 mm diameter hole, and its surface was observed by SEM. For SEM observation, a platinum coating with a thickness of 10 nm was applied to prevent charging. Fig. 30 shows the SEM images. Fig. 30(a) corresponds to the compressed region, and Fig. 30(b) corresponds to the uncompressed region (corresponding to the hole area in the aluminum block). Both regions were observed at 10000× magnification and an accelerating voltage of 3 kV. These results confirmed that the porous structure of the mixed cellulose ester membrane was collapsed using the hydraulic flask press and aluminum block. In contrast, the porous membrane portion corresponding to the hole in the aluminum block was not collapsed, and the porous structure could still be observed.

### 10-5. Transfer of a compressed porous membrane/graphene laminated body

Next, to determine whether the mixed cellulose ester membrane with compressed porous structures could function as a transfer support, a laminated body on copper foil in which part of the porous structure had been collapsed after applying force was transferred onto a quartz glass substrate following the same procedure as in Example 2. As a result, the transfer was successfully achieved, demonstrating that it still functioned as a transfer support even after partial collapse of the porous regions.

### 10-6. Electrochemical measurement using laminated electrodes comprising compressed porous membrane/graphene film

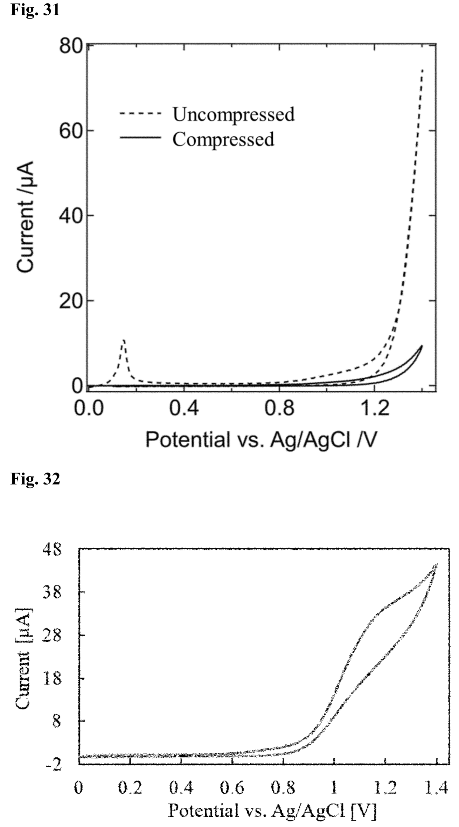

Porous mixed cellulose ester membranes were prepared following the same procedure as in 10-1, and transferred onto quartz glass substrates with chromium (10 nm) and silver (50 nm) deposited at the ends, in accordance with Example 2. Two such laminated bodies were prepared, and for one of them, a force of 1 ton was applied for 30 seconds using an aluminum block with a 4 mm diameter hole, following the procedure described in 10-3. Cyclic voltammetry was then performed using the measurement cell shown in Fig. 10 with both the compressed and uncompressed laminated electrodes, in 0.15 mol/L phosphate-buffered solution (PBS) at pH 7.4 over a potential range of 0-1.4 V vs. Ag/AgCl, with a scan rate of 0.1 V/s. The reference and counter electrodes were the same as those used in Example 4. The measurement results are shown in Fig. 31. In the uncompressed laminated electrode, an oxidation peak was observed at 0.15 V vs. Ag/AgCl, which was attributed to the oxidation of the silver in the contact electrode. Thus, it was found that in the uncompressed laminated electrode, the electrolyte solution (PBS) infiltrated laterally to the contact electrode, affecting the measurement results. In contrast, with the laminated electrode comprising compressed porous structures, a voltammogram without the silver oxidation peak of the contact electrode was obtained, demonstrating that the porous structures outside the measurement area could be compressed without damaging the graphene, thereby preventing lateral infiltration of the electrolyte solution.

Thus, it was found that by compressing the porous structure, the electrode area could be defined while preventing the solution from contacting the contact electrode. Since there was almost no damage to the graphene beneath the compressed region, this also provides an advantage in terms of charge transport to the electroactive region in contact with the electrolyte solution. By designing compressed and uncompressed portions, it is considered possible to fabricate arrays of electroactive regions through a simple process. Therefore, this method is also considered to have advantages from the viewpoint of fabricating biochips for multi-analyte testing.

### (Example 11. Immunoassay using a laminated electrode comprising a porous membrane/graphene film prepared by the solution-casting method) 11-1. Fabrication of laminated electrode and ECL immunoassay

Following the same procedure as in Example 9, a porous mixed cellulose ester membrane was formed on graphene grown on copper, using an applicator gap height of 50 µm. A force of 1 ton was then applied for 30 seconds using an aluminum block with a 4 mm diameter hole, in the same manner as described in Example 10. This laminated electrode was transferred onto a quartz glass substrate with Au/Cr deposition, as in Example 2. An ECL immunoassay for CEA was conducted using this laminated electrode following the procedure described in Example 6.

### 11-2. Results

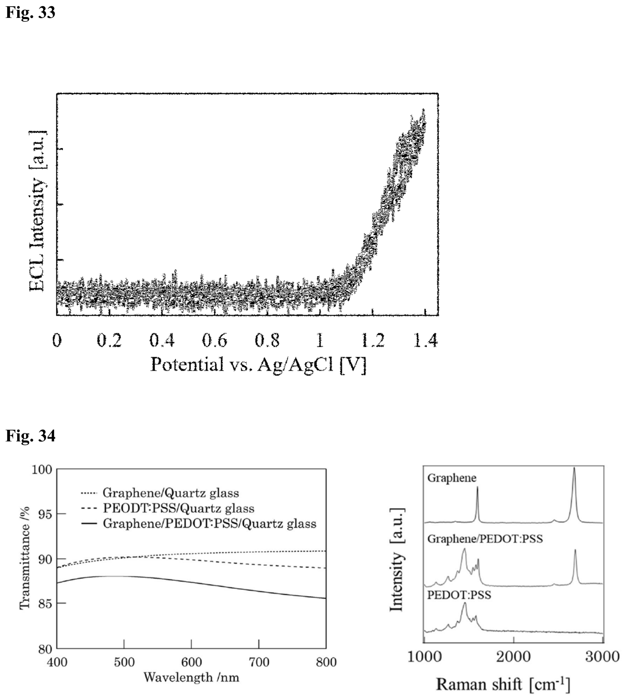

The measurement results are shown in Fig. 32 (current-potential curve) and Fig. 33 (ECL intensity-potential curve). An increase in current was observed around 0.8 V at the graphene electrode, confirming the oxidation of tripropylamine at the electrode. An increase in ECL intensity was also observed around 1.0 V. These results demonstrated that laminated electrodes comprising mixed cellulose ester porous membrane/graphene film fabricated by the solution-casting method were applicable as electrodes for ECL immunoassays.

### (Example 12. Graphene transparent electrode with PEDOT:PSS as a conductive additive)

Since graphene is a one-atom-thick material, it has a higher resistance than conventional electrodes such as platinum used in ECL measurements, and resistance values are likely to vary among samples. The electrode resistance may affect the emission intensity in ECL measurements. Therefore, in order to use graphene transparent electrodes as lower-resistance electrodes, PEDOT:PSS, which is also a transparent electrode material like graphene, was employed.

In this example, with a view to applying CVD-grown graphene as an electrode for electrochemiluminescence immunoassays, graphene and PEDOT:PSS were laminated, and the electrochemical characteristics thereof were evaluated in order to suppress variations in ECL emission intensity in the Ru(bpy)₃Cl₂/TPrA system used in bioanalysis.

### 12-1. Fabrication of graphene/PEDOT:PSS electrodes

Graphene/PEDOT:PSS electrodes were fabricated as follows. A 20 mm × 20 mm quartz glass substrate was cleaned in the same manner as the copper substrate used for graphene growth. Then, 5 nm of Cr and 50 nm of Au were deposited at the ends of the substrate. PEDOT:PSS (Sigma-Aldrich, Cat. No. 739316-25G) was dropped onto the substrate and spin-coated. The spin coating was performed by ramping the speed to 2500 rpm over 30 seconds, maintaining it for 60 seconds, and then stopping over 30 seconds. The film was then baked at 140°C for 1 hour to complete the PEDOT:PSS electrode. A monolayer graphene film was then transferred onto the surface of the PEDOT:PSS electrode using the conventional PMMA-assisted transfer method. After transferring the PMMA/graphene laminated film onto the PEDOT:PSS electrode, the film was thoroughly dried and then immersed in acetone to remove the PMMA, thereby obtaining the graphene/PEDOT:PSS electrode.

### 12-2. Transmittance and Raman spectroscopy

The left panel of Fig. 34 shows the transmittance spectra of the fabricated electrodes. The graphene electrode exhibited stable high transmittance of about 90%, whereas the graphene/PEDOT:PSS electrode showed decreasing transmittance toward longer wavelengths, appearing bluish in color. Focusing on the transmittance at 620 nm, the emission wavelength of the Ru(bpy)₃ complex used in this study, the values were approximately 90.6% for the graphene electrode, 89.7% for the PEDOT:PSS electrode, and 87.1% for the graphene/PEDOT:PSS electrode. Thus, the graphene/PEDOT:PSS electrode showed about a 2.6% decrease in transmittance compared to the PEDOT:PSS electrode. Since a single layer of graphene absorbs about 2.3%, this decrease was considered reasonable. Although the transmittance of the graphene/PEDOT:PSS electrode was about 3.5% lower than that of the graphene electrode, it still maintained high transparency. Therefore, it was demonstrated to be sufficiently applicable as a transparent electrode for ECL measurements.

The right panel of Fig. 34 shows the Raman spectra of graphene, PEDOT:PSS, and graphene/PEDOT:PSS. In the Raman spectrum of graphene, the characteristic 2D peak at 2680 cm⁻¹ and the G peak at 1590 cm⁻¹ were observed. The full width at half maximum of the 2D peak was 37 cm⁻¹, and the intensity ratio I(2D)/I(G) was 1.2. Since the 2D peak could be fitted with a single Lorentzian function, the CVD-grown graphene was determined to be monolayer graphene. No D peak near 1350 cm⁻¹, indicative of defects, was observed, confirming the growth and transfer of high-quality monolayer graphene. In the graphene/PEDOT:PSS electrode, the G peak of graphene was observed at 1598 cm⁻¹ and the 2D peak at 2690 cm⁻¹. It was also confirmed that the shorter wavelength side relative to 1590 cm⁻¹ matched the Raman spectrum of PEDOT:PSS alone. This indicates that PEDOT:PSS and graphene formed a laminated structure. Taken together with the transmittance results, it was concluded that the graphene/PEDOT:PSS electrode is a highly transparent electrode comprising high-quality graphene laminated with PEDOT:PSS.

### 12-3. Sheet resistance

The sheet resistance values of graphene, PEDOT:PSS, and graphene/PEDOT:PSS electrodes were measured. For this measurement, graphene (20 mm × 20 mm) transferred onto a 20 mm × 20 mm quartz glass substrate was used, and indium pellets were brought into contact with the four corners. Table 6 shows the sheet resistance values of each sample, measured using the four-point probe method.

**Table 6**

| Sample | Sheet resistance [Ω/sq] |
|---|---|
| Graphene | 925 |
| Graphene/PEDOT:PSS | 224 |
| PEDOT:PSS | 704 |

As shown in Table 6, both graphene and PEDOT:PSS individually exhibited high sheet resistance values, but the sheet resistance decreased significantly when laminated together. This reduction is attributed not only to the increased thickness of the electrode due to the lamination of graphene and PEDOT:PSS, but also to the fact that small cracks generated in the graphene during the transfer and other processes were compensated by the PEDOT:PSS layer.

### 12-4. CV measurement 1

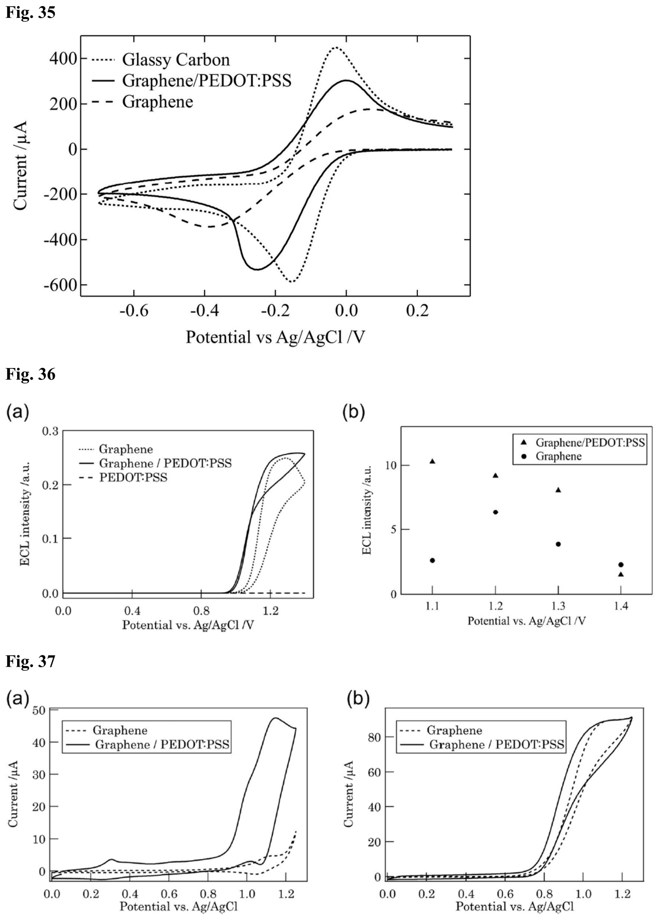

Fig. 35 shows the results of cyclic voltammetry (CV) measurements using graphene, graphene/PEDOT:PSS, and glassy carbon as electrodes in an aqueous solution of 0.1 mol/L KCl containing 5 mmol/L hexaammineruthenium. The scan rate was set at 0.1 V/s. Table 7 shows the peak-to-peak separation ΔEp, defined as the potential difference between the oxidation peak and the reduction peak.

**Table 7**

| | Graphene | Graphene/PEDOT:PSS | Glassy carbon |
|---|---|---|---|
| Oxidation peak [V] | 0.0681 | -0.0027 | -0.0030 |
| Reduction peak [V] | -0.3860 | -0.2493 | -0.1566 |
| *ΔEₚ* [mV] | 454 | 247 | 154 |

In a reversible system where the electrode reaction is sufficiently fast, ΔEp is 59 mV for a one-electron transfer reaction. However, when solution resistance or internal resistance of the electrode cannot be neglected, ohmic loss occurs due to the product of current and resistance, resulting in a larger ΔEp. Since the internal resistance of the glassy carbon electrode is negligibly small compared to the solution resistance, the difference in ΔEp between the glassy carbon electrode and the other two electrodes can be attributed to the internal resistance of the electrodes. Under the present measurement conditions, where relatively large currents flow, it was found that the graphene electrode alone exhibited large internal resistance, leading to significant ohmic loss. In contrast, the graphene/PEDOT:PSS electrode showed reduced ohmic loss, as supported by the decreased sheet resistance shown in Table 7, and ΔEp values closer to those of glassy carbon. Because the luminescence intensity in electrochemiluminescence (ECL) measurements is strongly correlated with the current value, a large internal resistance of the electrode can hinder achieving high luminescence intensity.

### 12-5. ECL measurement

Fig. 36(a) shows the results of ECL measurements by the CV method, and Fig. 36(b) shows the total luminescence intensity over 30 seconds after the onset of potential application, measured by chronoamperometry. The solution used was 0.15 mol/L phosphate-buffered solution (PBS) containing 10 mmol/L tripropylamine and 0.1 mmol/L Ru(bpy)₃Cl₂. When PEDOT:PSS alone was used as the working electrode for comparison, no luminescence was observed. This is considered to be due to the property of PEDOT:PSS to disperse in water, which caused it to redisperse into the measurement solution and lose its function as an electrode. On the other hand, in the graphene/PEDOT:PSS electrode, luminescence was confirmed, indicating that laminating graphene on PEDOT:PSS prevented PEDOT:PSS from redispersing into the measurement solution, thereby enabling its use in electrochemical measurements.

Furthermore, in the ECL measurements of the graphene/PEDOT:PSS electrode, luminescence was observed at lower potentials compared to the graphene electrode, indicating that lamination facilitated electrode reactions. The results in Fig. 36(b) also show that the graphene/PEDOT:PSS electrode produced a higher luminescence intensity than the graphene electrode, demonstrating that the presence of PEDOT:PSS contributed to the enhancement of ECL intensity.

### 12-6. CV measurement 2

Fig. 37(a) shows the CV measurement results for Ru(bpy)₃Cl₂, and Fig. 37(b) shows the CV measurement results for tripropylamine. In both cases, it was found that the graphene electrode laminated with PEDOT:PSS allowed current to flow at lower potentials. In particular, with respect to the reactivity of Ru(bpy)₃Cl₂, a significant enhancement in reactivity was observed in the presence of PEDOT:PSS. Therefore, the increase in ECL intensity of the graphene electrode due to PEDOT:PSS lamination can be attributed not only to the reduction of the electrode's internal resistance but also to the improvement in the reactivity of the ECL reactants.

Table 8 shows the average luminescence intensity and the standard error obtained when CV measurements were performed on five samples for each electrode. In addition, Table 8 shows, based on the ECL measurement results of Fig. 37(b), the coefficient of variation of the total luminescence intensity at 1.3 V.

**Table 8**

| | Graphene | Graphene/PEDOT:PSS |
|---|---|---|
| Average ECL intensity ± standard error (CV) | 26.1 ± 2.3 | 34.1 ± 1.3 |
| Coefficient of variation (CA) | 0.256 | 0.178 |

The average luminescence intensity measured by the CV method was approximately 1.3 times greater for the graphene/PEDOT:PSS electrode than for the graphene electrode. The variability of about 8.8% observed for the graphene electrode was reduced to about 3.8% for the graphene/PEDOT:PSS electrode. In CA measurements using five samples, the coefficient of variation calculated from the luminescence intensity at 1.3 V was also smaller for the graphene/PEDOT:PSS electrode. Accordingly, by laminating graphene with PEDOT:PSS, the ECL luminescence intensity was demonstrated to be increased and to enable more stable measurements with smaller error. This effect is considered to result from a reduction in the variability of the sheet resistance (internal resistance) due to the PEDOT:PSS layer, which reduced the variability in the electrochemical measurement results.

### (Example 13. Making a porous membrane/transparent conductive film laminated body transparent)

The laminated body composed of a porous membrane and a transparent conductive film was demonstrated to be applicable as a transparent conductive film with a separator. A separator is placed between the anode and cathode of a two-electrode system, allowing ion transmission while preventing direct contact between the electrodes. Typically, resin materials with a porous structure or nonwoven fabrics are used as separators. The porous membrane/graphene laminated body in this example was demonstrated to be applicable as a transparent electrode with a separator. Furthermore, it is considered that by laminating an additional graphene film on the surface of the porous membrane of the porous membrane/graphene laminated body, an ultrathin electrochemical cell with high transparency can be fabricated.

For instance, when fabricating a thin electrochromic device, two electrodes consisting of an anode and a cathode must be placed facing each other. By inserting a separator between the electrodes, internal short-circuiting can be prevented, and the necessary laminated structure can be achieved. However, in order to reversibly control the transparent (bleached) state and the colored state, the separator itself must be transparent. In general, porous materials appear white because they scatter light, even for wavelengths at which light absorption does not occur.

In this example, an attempt was made and it was demonstrated to render the device transparent by matching the refractive index of the separator placed between graphene electrodes with that of the electrolyte solution impregnated into the separator.

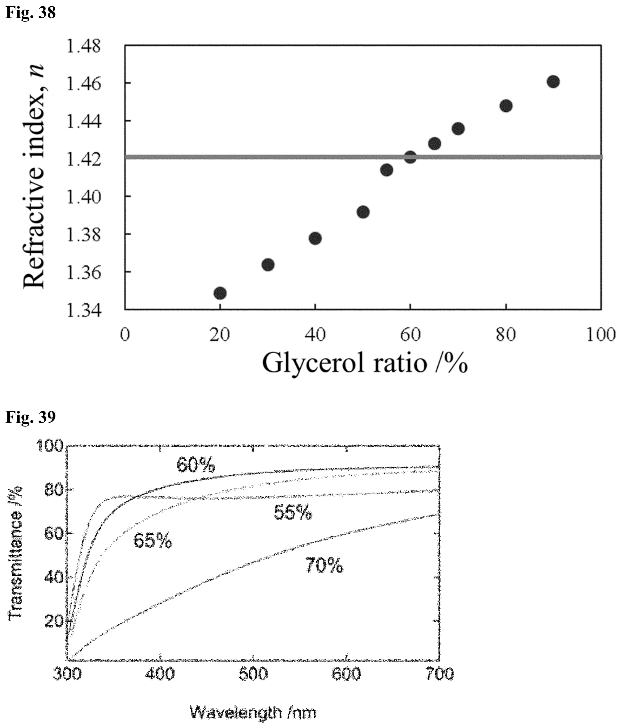

Specifically, to match the refractive indices of the electrolyte solution and the separator, polyvinylidene fluoride (PVDF) was selected as the separator, and a mixed solvent of water and glycerol was used as the solvent of the electrolyte solution. The refractive index of water is 1.33, while that of glycerol, which is miscible with water, is 1.47. Since the refractive index of PVDF is 1.41, which falls between those of the two solvents, PVDF was selected as the separator. First, to determine the optimal mixing ratio of water and glycerol in the electrolyte solution to be impregnated into PVDF, the dependence of the refractive index of water/glycerol mixed solvents on the glycerol ratio was investigated. The refractive index was measured using a digital refractometer (Hanna Instruments, model HI96800). Fig. 38 shows the dependence of refractive index on the glycerol ratio of the mixed solvent. As the glycerol ratio increased, the refractive index of the mixed solvent increased, and at a water-to-glycerol ratio of 4:6, the value approached the refractive index of PVDF (1.42).

Next, a commercially available porous PVDF membrane (GVS, product number 1213992) was immersed in methanol for 30 seconds for hydrophilization treatment, and then impregnated with various ratios of glycerol/water mixed solutions containing 0.1 M potassium chloride. Transmittance measurements were performed with the membrane sandwiched between two glass slides (Matsunami Glass Ind., Ltd., product number S1111). Fig. 39 shows the transmittance spectra. The PVDF membrane impregnated with a 0.1 M potassium chloride solution of water/glycerol at a ratio of 4:6 exhibited the highest transmittance. Since this value was equivalent to the transmittance of the glass slides alone, the difference from 100% was attributable to reflection at the air-glass interface, and it was confirmed that essentially no scattering occurred in the PVDF layer impregnated with the solution, so that the transmittance of this layer was regarded as being substantially 100%.

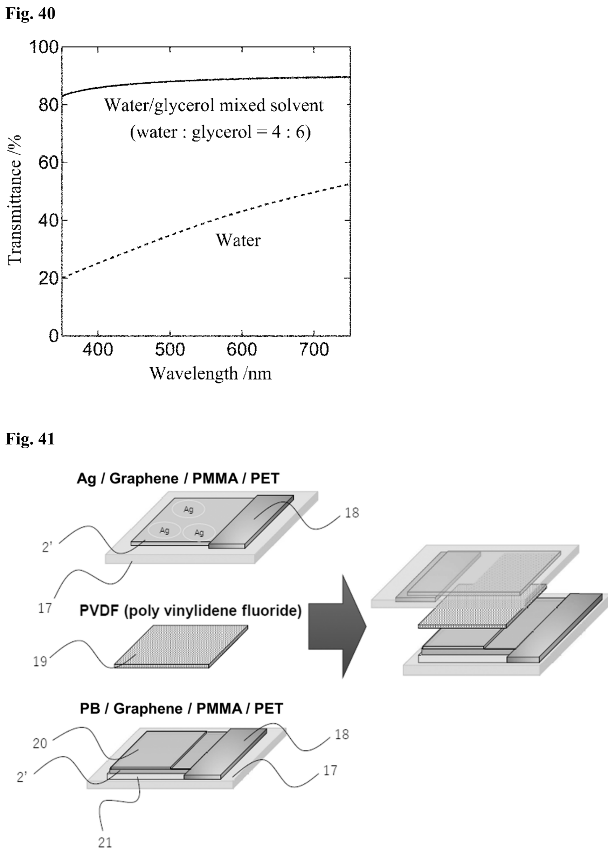

Subsequently, following the same procedure as in Example 9, a PVDF/graphene laminated electrode was fabricated by the solution-casting method using an applicator with a gap height of 100 µm. The PVDF porous-membrane-forming solution was prepared by dissolving PVDF (Sigma-Aldrich, product number 347078) at 12 wt% in a 1:1 mixed solvent of acetone and dimethylformamide (DMF). The fabricated PVDF/graphene/quartz glass substrate laminated body was immersed in methanol for hydrophilization treatment, followed by impregnation with water or a water/glycerol solution, and covered with a cover glass for transmittance measurements. Fig. 40 shows the transmittance spectra. While the transmittance at 550 nm was 39% for the laminated body impregnated with water, it reached 89% for the laminated body impregnated with a water/glycerol solution at a 4:6 weight ratio, thereby demonstrating that transparency of the PVDF porous membrane/graphene laminated body was successfully achieved.

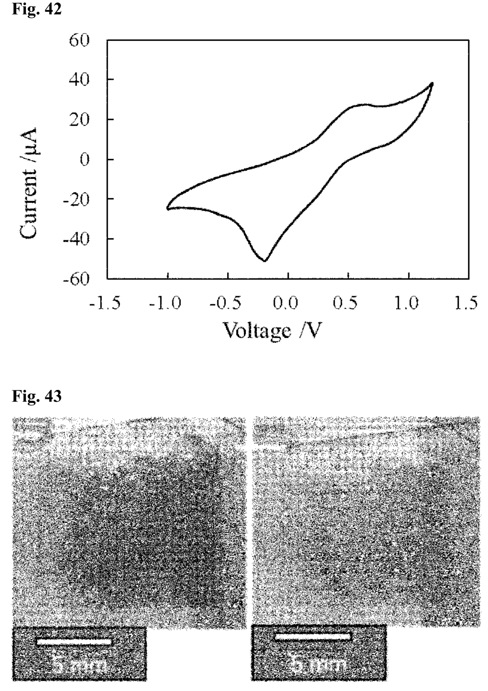

Next, a film-type electrochromic device was fabricated using two graphene electrodes with PVDF as a separator and a mixed solution of water and glycerol at a weight ratio of 4:6 as the electrolyte solution. Prussian blue (PB) was employed as the electrochromic material. Specifically, the electrochromic device was fabricated as follows. First, CVD graphene was transferred onto a PET substrate by thermocompression using PMMA as an adhesive, thereby obtaining a graphene electrode composed of a graphene film/PMMA/PET substrate. Two of the fabricated graphene electrodes were then used to prepare a PB-deposited graphene electrode and a silver-deposited graphene electrode, as described below. Using a solution containing 5 mmol/L ferric chloride (FeCl₃), 5 mmol/L potassium ferricyanide, 0.1 mol/L hydrochloric acid, and 0.1 mol/L potassium chloride, PB was electrodeposited onto the graphene electrode by applying a current density of -20 µA/cm² for 5 minutes, thereby producing a PB-deposited graphene electrode. For the counter electrode, it was necessary to induce a redox reaction of opposite polarity and equivalent charge to that of PB, so silver was electrodeposited to utilize the silver/silver chloride redox couple. For silver deposition onto the graphene electrode, a solution containing 0.1 mol/L sodium nitrate and 1 mmol/L silver nitrate was used, and a current density of -100 µA/cm² was applied for 4 minutes. The PB-deposited graphene electrode (oxidized/colored state) and the silver-deposited graphene electrode (reduced, Ag) were then assembled with a PVDF porous membrane as a separator. The PVDF was impregnated with a water/glycerol (4:6) solution containing 0.1 mol/L potassium chloride as the electrolyte solution. The voltage applied between the two electrodes (with the silver-deposited electrode as reference) was varied between -1.0 V to 1.2 V (starting from 0 V), and the corresponding current and color changes of the device were observed. Fig. 41 shows a schematic diagram of the fabricated electrochromic device. Fig. 42 shows the current-voltage curve, and it was confirmed that the color changed from blue (colored state, Fig. 43 left) to transparent (bleached state, Fig. 43 right) by reduction of PB (concurrent with oxidation of silver to silver chloride), and from transparent to blue by oxidation of PB (concurrent with reduction of silver chloride) (Fig. 43).

The method of matching the refractive indices of the electrolyte solution and the porous membrane is also considered to be effective in applications utilizing ECL, including ECL immunoassays. For example, if the refractive index of the electrolyte solution used in an ECL immunoassay is adjusted to match that of the mixed cellulose ester membrane, ECL emission may thereby be captured by a photodetector without scattering within the porous membrane. As a result, the boundary between the luminescent and non-luminescent regions can be maintained in a clearly defined state without blurring. Therefore, when associating multiple test items with emission positions on an analytical device, this approach is expected to contribute to the integration of multiple assays.

### Explanation of Reference Numerals

1 ... Laminated body (porous membrane/transparent conductive film)
1' ... Laminated body (porous membrane/transparent conductive film/transparent insulating substrate)
2 ... Transparent conductive film
2' ... Graphene film
3 ... Porous membrane
4 ... Transparent insulating substrate
5 ... Contact electrode
6 ... Membrane
7 ... Electrical conductor (counter electrode)
8 ... Conjugate pad
9 ... Sample pad
10 ... Absorbent pad
11 ... Biochip
12 ... Photodetector
13 ... Reference electrode
14 ... Image sensor
15 ... Metal catalyst substrate
16 ... Electrolyte solution
17 ... PET substrate
18 ... Gold (Au)
19 ... PVDF membrane
20 ... Prussian Blue (PB)
21 ... PMMA

## Claims

1. A laminated body comprising a transparent conductive film and a porous membrane laminated on the transparent conductive film,
wherein the porous membrane is permeable to an electrolyte solution.

2. The laminated body according to claim 1,
wherein the porous membrane has pores with an average pore diameter of 20 nm or more.

3. The laminated body according to claim 1,
wherein the pore area ratio on the porous membrane surface is 20% or more.

4. The laminated body according to claim 1,
wherein the porous membrane has a film thickness of 100 nm or more.

5. The laminated body according to claim 1,
further comprising a transparent insulating substrate,
wherein the porous membrane is laminated on a first surface of the transparent conductive film, and the transparent insulating substrate is laminated on a second surface of the transparent conductive film.

6. The laminated body according to claim 5,
wherein the laminated body further comprises an electrical conductor, the electrical conductor being laminated on a surface of the porous membrane opposite to the surface on which the transparent conductive film is laminated, or on a surface of the transparent insulating substrate on the same surface as the surface on which the transparent conductive film is laminated.

7. The laminated body according to claim 5, further comprising a transparent insulating substrate,
wherein the porous membrane has a compressed structure at least in part.

8. The laminated body according to claim 5,
wherein the transparent insulating substrate is coated with a transparent conductive additive.

9. The laminated body according to claim 1,
further comprising an electrical conductor,
wherein the transparent conductive film is laminated on a first surface of the porous membrane, and the electrical conductor is laminated on a second surface of the porous membrane.

10. The laminated body according to claim 1,
wherein the porous membrane is a membrane made of a material selected from the group consisting of nitrocellulose, cellulose acetate, polyethersulfone, polytetrafluoroethylene, polyamide, polyvinylidene fluoride, regenerated cellulose, polycarbonate, polypropylene, polyvinylidene chloride, aluminum oxide, glass fibers, quartz fibers, polymethyl methacrylate, polystyrene, polyethylene, polyethylene terephthalate, and ceramics, or a mixed membrane made of two or more materials selected from the group.

11. The laminated body according to any one of claims 1 to 10,
wherein the transparent conductive film is a carbon-based conductive film.

12. The laminated body according to any one of claims 1 to 10,
wherein the transparent conductive film is a graphene film.

13. A biochip comprising the laminated body according to any one of claim 5 to claim 9.

14. A kit comprising the biochip according to claim 11 and an electrochemiluminescent substance.

15. The laminated body according to claim 5, further comprising an additional transparent conductive film, an additional transparent insulating substrate, and an electrical conductor,
wherein the laminated body is laminated in the order of a transparent insulating substrate, transparent conductive film, porous membrane, transparent conductive film, and transparent insulating substrate, and
wherein the electrical conductor is disposed so as to be in contact with the transparent conductive film.

16. A method of using the laminated body according to claim 5 as a transparent electrode,
wherein a solvent having a refractive index substantially identical to that of the porous membrane is used.

17. A method for detecting a target compound by electrochemiluminescence immunoassay using the laminated body according to any one of claim 5 to claim 9 or the biochip comprising the laminated body according to any one of claims 5 to 7, the method comprising:
(a) binding the target compound with an electrochemiluminescent substance;
(b) capturing the target compound in the porous membrane of the laminated body with a specific binding substance for the target compound immobilized in the porous membrane; and
(c) after steps (a) and (b), applying a potential to the laminated body and measuring electrochemiluminescence.

18. A method for producing a laminated body in which a transparent insulating substrate, a carbon-based transparent conductive film, and a porous membrane are laminated in this order, the method comprising:
(a) forming a carbon-based transparent conductive film on a metal catalyst substrate by a chemical vapor deposition (CVD) method;
(b) further forming a porous membrane on the carbon-based transparent conductive film to produce a laminated body; and
(c) removing the metal catalyst substrate by etching and transferring the laminated body so that the carbon-based transparent conductive film is in contact with the transparent insulating substrate.

19. The method according to claim 18,
wherein, in step (b), a solvent for dissolving a polymer used to form the porous membrane is a mixed solvent comprising a good solvent and a poor solvent for the polymer.
